# EUROPEAN PATENT APPLICATION

(11) **EP 4 818 330 A1**
(43) Date of publication of application: **07.10.2026**
(21) Application number: 24896592.3
(22) Date of filing: 27.11.2024
(51) Int. Cl.: C12Q 1/6883, G01N 33/68, A61P 25/28

(54) **PHARMACEUTICAL PREPARATION FOR DIAGNOSING AND TREATING ALZHEIMER'S DISEASE**

(30) Priority: 01.12.2023 CN 202311635658
(71) Applicant: Shanghai East Hospital Tongji University, Shanghai 200120 (CN)
(72) Inventor: LI, Gang, Shanghai 200120 (CN)
(74) Representative: Huang, Liwei
(86) International application number: PCT/CN2024/135004
(87) International publication number: WO 2025/113506

(57) **Abstract**

The present invention relates to the field of biomedicine. Provided are a marker that can be used for early diagnosis of Alzheimer's disease, and the use thereof. Specifically provided is a marker for early diagnosis of Alzheimer's disease. The marker is a Maf1 gene or the protein rhereof. High expression of the Maf1 gene or the protein thereof in neuronal cells indicates that a subject is at high risk of developing Alzheimer's disease. The method provides a new diagnostic and therapeutic target for AD.

## Description

### Technical Field

The present disclosure belongs to the field of biomedicine and relates to a marker for early diagnosis of Alzheimer's disease and a pharmaceutical preparation for the treatment of the Alzheimer's disease.

### Background

Alzheimer's Disease (AD) is a progressive and irreversible degenerative brain disorder characterized by progressive dementia. The pathological changes of this disease are characterized by relatively extensive cerebral atrophy, also known as diffuse cerebral atrophy. With the extension of human lifespan and the increase in age, the incidence of this disease shows an upward trend, thus attracting increasing attention. Alzheimer's disease is clinically manifested as a gradual onset, with early symptoms including mild personality changes, intellectual decline, decreased ability of determination, lack of interest in surroundings, easy fatigue, nervousness, anxiety and irritability, sleep disturbances, headaches and dizziness. Subsequently, it manifests as: (1) memory impairment, primarily characterized by recent memory loss; (2) impairment in thinking and judgment; (3) personality changes; (4) emotional disorders: such as depression and reticence; (5) hallucinations and delusions: predominantly auditory hallucinations, more frequent at night; (6) loss of the ability to synthesize information obtained from visual, auditory, tactile and olfactory senses. The patients become unable to live independently, and as the condition further progresses, they cannot remember the names of their relatives or even recognize them, exhibit incoherent speech with stuttering, have poor appetite, show significant weight loss, and eventually become so physically weak that they are bedridden, incontinent, and often succumb to concurrent infections. The basic symptom of Alzheimer's disease is organic dementia, but various psychotic symptoms may also occur. Strecker et al. classified Alzheimer's disease into five types: simple dementia type, delirious-confused type, paranoid type, anxious-depressed type, and presenile type. One of the typical symptoms of the Alzheimer's disease is memory impairment, but the memory impairment caused by the Alzheimer's disease is different from ordinary insomnia and forgetfulness. The ordinary insomnia and forgetfulness is the loss of memory traces in the brain, manifested as the inability to recall previously perceived things, which is temporary forgetting, and the forgotten memory may be restored under suitable conditions. Moreover, ordinary patients with insomnia and amnesia generally maintain intact insight, i.e., the patients have insight into their abnormal mental manifestations, suffer from emotional distress, and voluntarily seek medical intervention. Their insomnia and amnesia symptoms can be improved through psychological adjustment or other relaxation methods. In contrast, patients with the Alzheimer's disease generally lack insight, are unable to recognize their own pathological manifestations and have the condition which is characterized by permanent amnesia. Furthermore, the patients completely fail to recall all events or experiences over a certain period. This is because memory impairment caused by the Alzheimer's disease often has an organic brain lesion basis.

The etiology of the AD disease is not yet fully clear, but several risk factors are known to contribute to its onset. Preventive measures targeting these risk factors should be able to achieve certain effects. The etiology is classified into the following categories: head trauma, educational level, family history of dementia, Down syndrome and the family history of Parkinson's disease, medical history, aluminum pots, etc. wherein boxers are prone to develop AD disease after repeated head trauma, known as Dementia Pugilistica (Punchdrunk syndrome). Studies have shown a significant increase in the incidence of the disease among individuals with a history of head trauma (RR is 1.8) (Monique M B, Breteler, Jules J. Claus, Cornelia M. Van Duijn, et al. Epidemiology of Alzheimer's Disease. Epidemiologic Reviews, 1992, 14: 59-82). Comparative studies have also found that head trauma accompanied by loss of consciousness is positively correlated with the onset of the AD disease. A history of head trauma within 10 years prior to the onset of the disease increases the susceptibility, and males are more commonly affected by trauma-induced cases. Previous medical history studies have shown that the Alzheimer's disease is associated with a history of thyroid disease. Therefore, both genetic and environmental factors can independently increase the risk of the AD disease, and it is conceivable that their combined effect may further contribute to the occurrence of the disease. Studies by Cornelia M et al. indicate that individuals with both head trauma and a family history of dementia have an OR value of 9.2.

The hippocampus is an important tissue of the central nervous system, which is mainly involved in advanced physiological functions such as learning, memory and emotion. Hippocampal neuronal damage may affect hippocampal function, eventually evolving into learning disabilities, memory dysfunction, depression, temporal lobe epilepsy, etc., which is one of the pathological features exhibited by the Alzheimer's disease. By inhibiting hippocampal damage and improving hippocampal neuroplasticity, the symptoms of the Alzheimer's disease can be alleviated to a certain extent.

Neuroinflammation also plays an important role in neurodegenerative diseases. Neuroinflammation is an inflammatory response occurring in the brain, which may be caused by a plurality of factors such as brain trauma, β-amyloid deposition, energy metabolism disorders, or even aging. Studies have shown that massive release of pro-inflammatory cytokines can damage neurons and lead to synaptic loss and neuronal death, which is associated with disease progression in patients and plays an important role in evaluating the condition of Alzheimer's disease.

Studies have found that decreased antioxidant capacity and accumulation of free radicals in patients with the Alzheimer's disease are important causes of neurotoxicity, thereby affecting learning and memory functions. SOD and GSH-PX are important antioxidant enzymes in the body. Their activities decrease in patients with learning and memory impairment, leading to the massive production of free radicals and subsequent damage to brain tissue function.

Currently, the clinical diagnostic criteria of Alzheimer's disease include: (1) clinical examination reveals dementia, supported by positive results from simple mental status tests [such as the Mini-Mental State Examination (MMSE), Blessed Dementia Scale, Hasegawa Dementia Scale (HDS), and Wechsler Adult Intelligence Scale] or other neuropsychiatric tests; (2) two or more cognitive dysfunctions; (3) progressive worsening of memory and other cognitive dysfunctions; (4) absence of consciousness disorders; (5) onset mostly after the age of 65; (6) exclusion of other brain lesions that may cause progressive memory and cognitive dysfunctions; (7) laboratory tests: normal lumbar puncture CSF, normal EEG or non-specific slow wave increase; (8) CT shows progressive cerebral atrophy, often prominent in the frontal lobe; (9) radioactive isotope cisternography reveals ventricular enlargement with rapid emptying; (10) early EEG manifestations include low amplitude, reduced α rhythm, gradually developing widespread θ waves and α wave disappearance, with later stages possibly showing burst or scattered δ waves, which may also be asymmetrical on both sides. It is generally believed that the definitive diagnosis of Alzheimer's disease relies only on pathological examination (neurofibrillary tangles, granulovacuolar degeneration and senile plaques within neurons). However, a detailed clinical course and relevant examinations to exclude other organic diseases causing dementia can still enable clinical diagnosis. The above diagnostic criteria are referred to as NINCDS-ADRDA, which is adopted by the United States and other countries (Epidemiology of Alzheimer's Disease; Wang Zhijin et al., Chinese Journal of Epidemiology 1998, (03), 173-176).

Early-onset AD is mainly associated with autosomal dominant inheritance, and late-onset AD has a high correlation with acquired factors. Studies have shown that high-risk factors for late-onset AD include hypertension, atherosclerosis, diabetes, smoking, metabolic syndrome, etc. (NAGAI M, DOTE K, KATO M, et al. Visit-to-visit blood pressure variability and Alzheimer's disease: links and risks [J]. J Alzheimers Dis, 2017, 59(2): 515-526.); its pathological manifestations mainly include intracellular neurofibrillary tangles (NFTs) and extracellular Aβ deposition. In addition, increased microglia in senile plaques are also observed (KENNEY K, IACONO D, ED LOW B L, et al. Dementia after moderate-severe traumatic brain injury: coexistence of multiple proteinopathies [J]. J Neuropathol Exp Neurol, 2018, 77(1): 50-63).

It is worth noting that AD is also a lethal neurodegenerative disease. The pathological features of AD include senile plaques formed by β-amyloid (Aβ) aggregation in the brain, neurofibrillary tangles (NFTs) formed by hyperphosphorylated Tau protein aggregation, chronic inflammatory responses, synaptic loss and neuronal death, neurotransmitter deficits, oxidative stress, inflammation, gene mutations, excitatory neurotoxins, etc. (Paasila, P.J., et al., Synapses, Microglia, and Lipids in Alzheimer's Disease. Front Neurosci, 2021. 15: p. 778822.2022 Alzheimer's disease facts and figures. Alzheimers Dement, 2022. 18(4): p. 700-789.). Recent studies have also shown that in many Alzheimer's patients without senile plaques and neurofibrillary tangles in the brain, the morphology and function of synapses have significantly degenerated, and even some neurons have died. Compared with amyloid plaque formation or neuronal loss, synaptic loss is the most obvious morphological correlate of cognitive impairment in early AD, and synaptic degeneration has become a biological marker of early AD (Lleó, A., et al., Changes in Synaptic Proteins Precede Neurodegeneration Markers in Preclinical Alzheimer's Disease Cerebrospinal Fluid. Mol Cell Proteomics, 2019. 18(3): p. 546-560.). A recent study also found that transcriptional regulation coordinates and regulates synaptic plasticity (Michels, A.A., et al., mTORC1 directly phosphorylates and regulates human MAF1. Mol Cell Biol, 2010. 30(15): p. 3749-57.). In addition, studies have found that after AD, many transcriptional regulators in the brain undergo significant changes (Mathys, H., et al., Single-cell transcriptomic analysis of Alzheimer's disease. Nature, 2019. 570(7761): p. 332-337.). However, the roles of these transcription factors after AD remain unclear. Studies have also shown that NMDA receptors are ligand-gated ion channels that play a critical role in memory formation. Presynaptic NMDA receptors regulate glutamate release and synaptic plasticity, and they may control spontaneous release independently of Mg²⁺ and Ca²⁺, whereas evoked release is dependent on Mg²⁺. The activation of extrasynaptic NMDA receptors leads to long-term depression (LTD), spine shrinkage and synaptic loss, and induces glutamate excitotoxicity, thereby causing AD (WANG R, REDDY P H. Role of glutamate and NMDA receptors in Alzheimer's disease [J]. J Alzheimers Dis, 2017, 57(4): 1041-1048). Therefore, NMDA is one of the targets for treating AD.

Maf1 is a highly conserved protein in humans, rats, mice and lower animals such as Drosophila and silkworms. Maf1 was first discovered in *Saccharomyces cerevisiae* and can be a transcription regulator of RNA polymerase III. It plays an essential role in tumors, glucose metabolism and lipid metabolism by activating the PTEN pathway. In recent years, it has also been found that Maf1 binds to RNA polymerase III and blocks transcription initiation and elongation regulated by RNA polymerase III. Moreover, it was found that it was capable of regulating the activity of RNA polymerase I and RNA polymerase II. It is worth noting that the activity of Maf1 in yeast is regulated by various environmental factors, for example, nutrient-limited environments induced by rapamycin or glucose deprivation, as well as DNA damage and yeast secretion-deficient environments. All of these factors affect the activity of Maf1. The activity of human Maf1 is altered in the context of DNA damage signals and rapamycin. The study also found that Maf1 can regulate the function of mitochondrial in yeast cells. In addition to affecting mitochondrial function, Maf1 is also able to regulate glucose metabolism, autophagy response, lipid metabolism, etc., thereby playing roles in tumorigenesis, reproductive capacity, obesity, growth and development, life expectancy, etc. It is also highly expressed in the central nervous system, particularly in the hippocampus and cortex. In addition, the latest research has demonstrated that Maf1 can negatively regulate the growth of hippocampal neuron dendrites through the PI3K-AKT-mTOR signaling pathway and negatively regulate dendritic spine growth to affect learning and memory abilities in mice (Chen, K., et al., Maf1 regulates dendritic morphogenesis and influences learning and memory. Cell Death Dis, 2020. 11(7): p. 606.). The report also confirms the neuroprotective effect of Maf1 on the survival period after RGC injury and provides a potential therapeutic strategy for traumatic optic neuropathy. The result shows that Maf1 becomes a hotspot in the central nervous system. Therefore, exploring the regulation of neuronal synapse morphology and potential functions by Maf1, as well as identifying small molecules that modulate Maf1, is of great significance for the diagnosis and treatment of Alzheimer's disease.

### Summary

In the present disclosure, under the pathological state of AD, the expression of the transcriptional regulatory protein factor Maf1 increases. Maf1 binds to the promoter region of Grin1, thereby regulating the expression of NMDAR1, further modulating calcium homeostasis, altering the morphological structure and quantity of neuronal dendritic spines, and ultimately affecting synaptic function. The present disclosure reveals that the Maf1-NMDAR1 pathway causes synaptic and calcium defects, and discovers small molecule compounds capable of acting on this pathway, thereby providing a novel diagnostic and therapeutic approach for AD. To this end, the present disclosure is completed.

In a first aspect, the present disclosure provides a marker for early diagnosis of Alzheimer's disease, wherein the marker is a Maf1 gene or a protein thereof, and the high expression of the Maf1 gene or the protein thereof in neuronal cells indicates that the test subject is at a high risk of the Alzheimer's disease.

In a second aspect, the present disclosure provides a reagent or kit for early diagnosis of Alzheimer's disease, wherein the reagent or kit comprises a reagent capable of detecting the expression of a Maf1 gene or a protein thereof.

In a third aspect, the present disclosure provides use of a Maf1 gene or a protein thereof in the preparation of a product for early diagnosis of Alzheimer's disease, wherein the use is to determine whether the patient is at a high risk of Alzheimer's disease based on the expression of the Maf1 gene or the protein thereof in neuronal cells.

Further, the Maf1 gene or the protein thereof is highly expressed in neuronal cells, and the test subject is determined to be at a high risk of Alzheimer's disease.

Further, the product comprises a chip and a kit.

In a fourth aspect, the present disclosure provides an inhibitor for inhibiting the expression of a Maf1 protein.

Further, the inhibitor includes, but is not limited to, chemically synthesized substances, naturally occurring compounds and/or biosynthesized substances.

Further, the inhibitor comprises one or more of a compound, a protein, an enzyme, a nucleic acid, a hormone, an antibody and a cytokine.

Further, the inhibitor has a structure represented by the following Formula I: (ETHYL4-[3-(3-{[2-(AZEPAN-1-YL)ETHYL]CARBAMOYL}PIPERIDIN-1-YL)-3-OXOPROPYL]-3, 5-DIMETHYL-1H-PYRROLE-2-CARBOXYLATE)

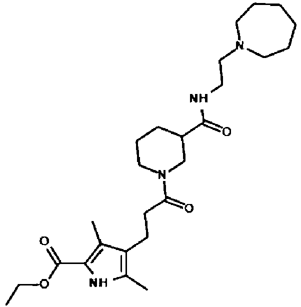

In a fifth aspect, the present disclosure provides a promoter for promoting the expression of a Maf1 protein.

Further, the promoter includes, but is not limited to, chemically synthesized substances, naturally occurring compounds and/or biologically synthesized preparations.

Further, the promoter comprises one or more of a compound, a protein, an enzyme, a nucleic acid, a hormone, an antibody and a cytokine.

In a sixth aspect, the present disclosure provides use of a promoter for promoting the expression of a Maf1 gene or a protein thereof in the preparation of an animal model, wherein the promoter is an experimental preparation.

Further, the animal model may be an Maf1 overexpression model, a cognitive dysfunction model, etc.

In a seventh aspect, the present disclosure provides use of an inhibitor for inhibiting the expression of a Maf1 gene or a protein thereof in the preparation of a medicament for treating AD disease.

Further, the inhibitor includes, but is not limited to, chemically synthesized substances, naturally occurring compounds and/or biosynthesized substances.

Further, the inhibitor comprises one or more of a compound, a protein, an enzyme, a nucleic acid, a hormone, an antibody and a cytokine.

Further, the inhibitor has a structure represented by the following Formula I: (ETHYL4-[3-(3-{[2-(AZEPAN-1-YL)ETHYL]CARBAMOYL}PIPERIDIN-1-YL)-3-OXOPROPYL]-3, 5-DIMETHYL-1H-PYRROLE-2-CARBOXYLATE)

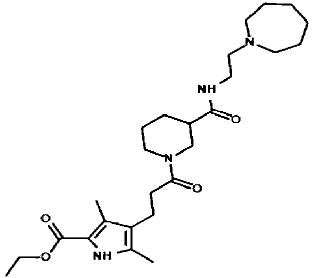

In an eighth aspect, the present disclosure provides a pharmaceutical preparation for treating AD disease, wherein the pharmaceutical preparation comprises an inhibitor for inhibiting the expression of a Maf1 gene or a protein thereof, and the pharmaceutical preparation further comprises a pharmaceutically acceptable carrier thereof.

Further, the inhibitor has a structure represented by the following Formula I: (ETHYL4-[3-(3-{[2-(AZEPAN-1-YL)ETHYL]CARBAMOYL}PIPERIDIN-1-YL)-3-OXOPROPYL]-3, 5-DIMETHYL-1H-PYRROLE-2-CARBOXYLATE)

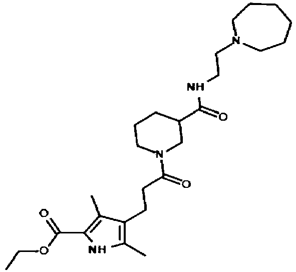

### Beneficial effects

In the present disclosure, it is discovered that Maf1 regulates the expression of NMDAR1 by binding to the promoter region of Grin1, thereby further regulating calcium homeostasis and synaptic remodeling in neurons. Therefore, the present disclosure reveals that the Maf1-NMDAR1 pathway causes synaptic and calcium defects, and provides a novel diagnostic and therapeutic target for AD.

### Brief Description of the Drawings

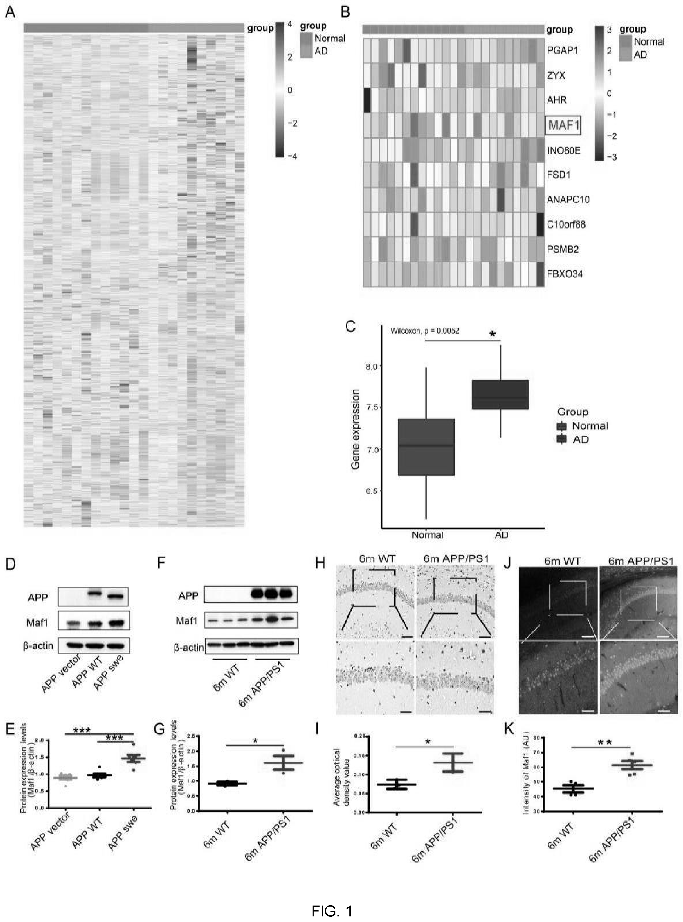

FIG. 1 shows the increased Maf1 expression in AD patients and APP/PS1 mice. (A) Heatmap of differentially expressed genes between AD and normal human brain in GSE5281 dataset. (B) Heatmap of Maf1 expression in normal individuals and AD patients in the dataset. (C) Compared with corresponding non-dementia control groups (n=13 and n=10, respectively), the expression of Maf1 is elevated in the human brain dataset of AD patients. (D-E) Quantitative Western blot analysis of primary hippocampal neurons with increased Maf1 in APPswe virus-transfected hippocampus compared with Vector, an APP WT virus. WB electrophoresis diagram and statistical diagram. (F-G) Comparison of Maf1 protein levels in hippocampal neurons of 6-month-old APP/PS1 mice with WT mice. n=4 mice per group. (H-I) shows the immunohistochemical results of Maf1 in 6-month-old APP/PS1 mice. n=4 mice per group. (J-K) show the immunofluorescence results of Maf1 in 6-month-old APP/PS1 mice. The data are presented as mean±SEM. *p<0.05; **p<0.01; ***p<0.001.

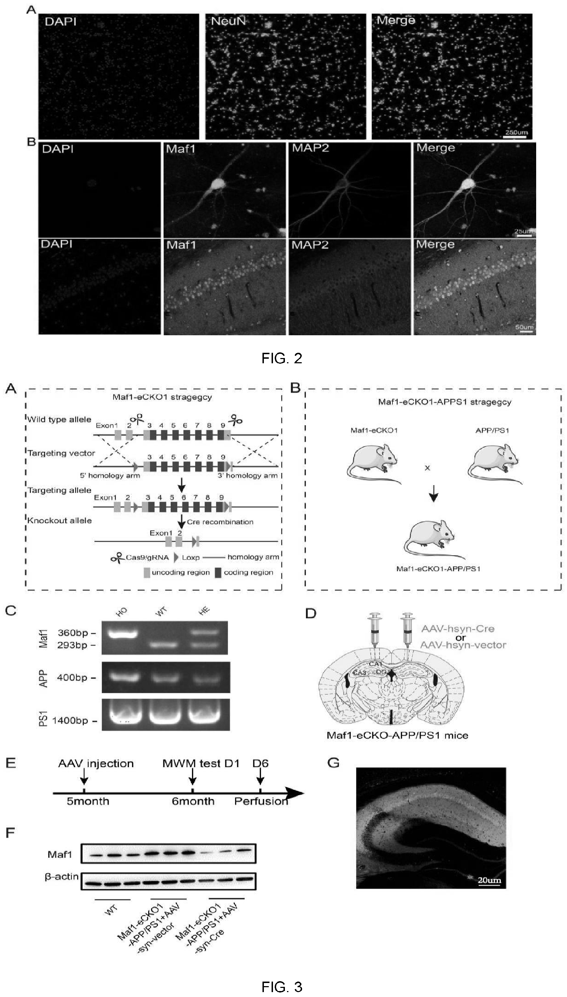

FIG. 2 shows the expression of Maf1 in hippocampal primary neuron dendrites. (A) The purity of primary neurons NeuN was observed by laser confocal microscopy. Scale: 250 µm. (B) Immunofluorescence staining of Maf1 and MAP2 in hippocampal neurons and brain slices cultured for 14 days from 6-month-old mice was observed using a laser scanning confocal microscope. Scale: above, 25 µm; below, 50 µm.
FIG. 3 shows the construction of Maf1-eCKO1-APP/PS1 transgenic knockout mice. (A) A schematic diagram of a design strategy for constructing a Maf1-eCKO1 conditional knockout mouse. Based on the principle of homologous recombination, the Maf1 gene is modified using flox through homologous recombination of fertilized eggs. (B) A schematic diagram of crossing between Maf1-eCKO1 and APP/PS1 mice. (C) PCR identification of DNA from Maf1-eCKO1-APP/PS1 hybrid mice. (D) A schematic diagram of stereotactic injection of adeno-associated virus. (E) A schematic diagram of experimental design. The Morris water maze (MWM) test is performed 1 month after virus injection. (F) After injection of the AAV-Cre virus, Western blot is used to confirm successful knockdown of Maf1 and corresponding reduction of NMDAR1 in the Maf1-eCKO1-APP/PS1 transgenic mice. (G) Fluorescence image of the hippocampus of Maf1-eCKO1-APP/PS1 hybrid mice injected with the AAV-Cre virus. Scale: 20 µm.

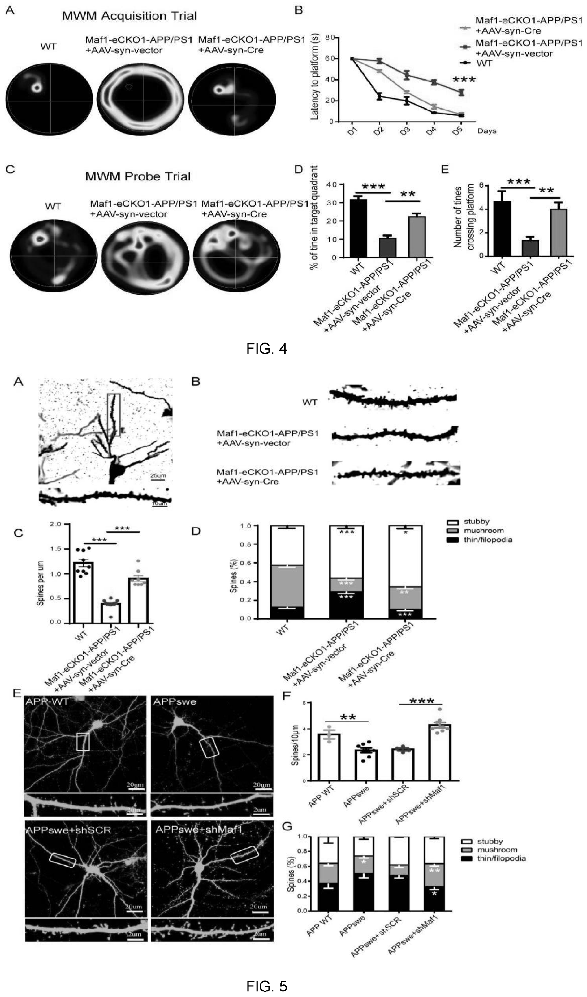

FIG. 4 shows that the conditional knockout of Maf1 improves learning and memory functions in AD mice. (A) A representative diagram of water maze trajectories of mice in each group on day 5 of the acquisition training phase. (B) The escape latency to find the hidden escape platform was measured during days 1-5 of the acquisition phase. (C) Characterization of water maze trajectories of mice in each group during the spatial exploration test phase on day 6. (D-E) The percentage of time spent in the target quadrant and the number of crossings over the target platform area during the spatial probe phase in three groups of mice. n = 10 mice per group. The data are presented as mean±SEM. *p < 0.05; **p < 0.01; ***p < 0.001.
FIG. 5 shows that the conditional knockout of Maf1 in hippocampal neurons promotes the maturation of neuronal dendritic spines in AD. (A) Golgi staining of mouse hippocampal neurons. The dendritic spines of hippocampal neurons in mice after viral injection are analyzed. Neurons are selected under a 40x microscope and the number of dendritic spines is analyzed under a 100x microscope. (B) High-magnification expression of dendritic spines in hippocampal neurons of WT mice and Maf1-eCKO1-APP/PS1 mice injected with AAV-syn-Cre-GFP and AAV-syn-GFP vectors after Golgi staining. Scale: 10 µm. (C) Statistical analysis of the density of dendritic spines of neurons in the three groups of mice hereinabove. (D) A statistical chart of classification percentages of dendritic spines of the three groups of mice hereinabove. n = 7-10 neurons per group. (E) Fluorescence microscopy GFP fluorescence of primary hippocampal neurons after transfection with shSCR and shMaf1 plasmids. The enlarged image of the white box is shown as follows. Scale: above, 20 µm; below, 2 µm. (F) Statistical analysis of the density of dendritic spines in cultured neurons. (G) A statistical chart of the percentage classification of dendritic spines in cultured neurons. n = 3-6 neurons per group. The data are presented as mean±SEM. *p < 0.05; **p < 0.01; ***p < 0.001.

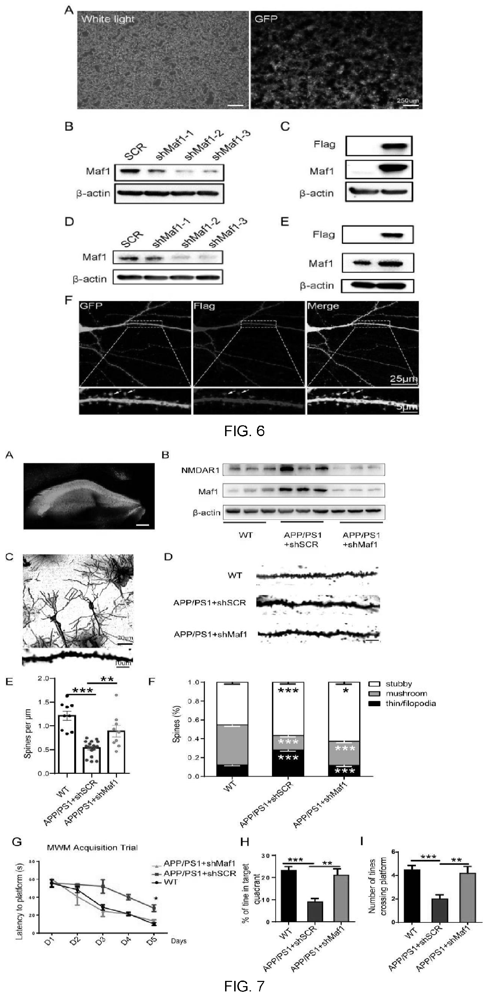

FIG. 6 shows the construction and validation of the shRNA virus for the target gene Maf1 interference and the full-length virus in cell lines and primary neurons. (A) Viruses are transfected into a 293T cell line. At 72 h post-transfection, the green fluorescence intensity of GFP is relatively high. (B-C) Western blotting is performed to determine the knockdown effect of shRNA on Maf1 and the overexpression effect of Maf1 in the 293T cell line. (D-E) Western blotting is performed to determine the knockdown effect of shRNA on Maf1 in primary neurons and the impact of Maf1 overexpression. (F) Neurons are transfected with Flag-tagged Maf1 overexpression plasmids. After culture for 14 days, immunofluorescence staining is performed.
FIG. 7 shows that the knockout of endogenous Maf1 in hippocampal neurons improves cognitive function in AD.
   (A) One month after virus injection, AAV-GFP green fluorescence expression is observed in the hippocampal slices of mice. (B) Western blotting is performed to determine the expression of Maf1 in the hippocampus of AD mice injected with AAV-shMaf1. (C) Golgi staining of mouse hippocampal neurons. Neurons are selected under 40x microscope, and the morphology and number of dendritic spines are analyzed under 100x microscope. (D) Representative high-magnification images of Golgi-stained dendritic spines of hippocampal neurons in WT mice and APP/PS1 mice injected with AAV-syn-shSCR and AAV-syn-shMaf1. Scale: 10 µm. (E) Statistical analysis of the density of dendritic spines of the neurons of the three groups of mice hereinabove. (F) A statistical chart of the classification percentage of dendritic spines of the 3 groups of mice hereinabove. n = 7-10 neurons per group. (G) The latency to find the escape platform is measured on days 1-5 of the training phase. (H-I) The percentage of time spent in the target quadrant and the number of crossings across the target platform area during the spatial probe phase in the three groups of mice. n = 10 mice per group. The data are presented as mean±SEM. *p < 0.05; **p < 0.01; ***p < 0.001.

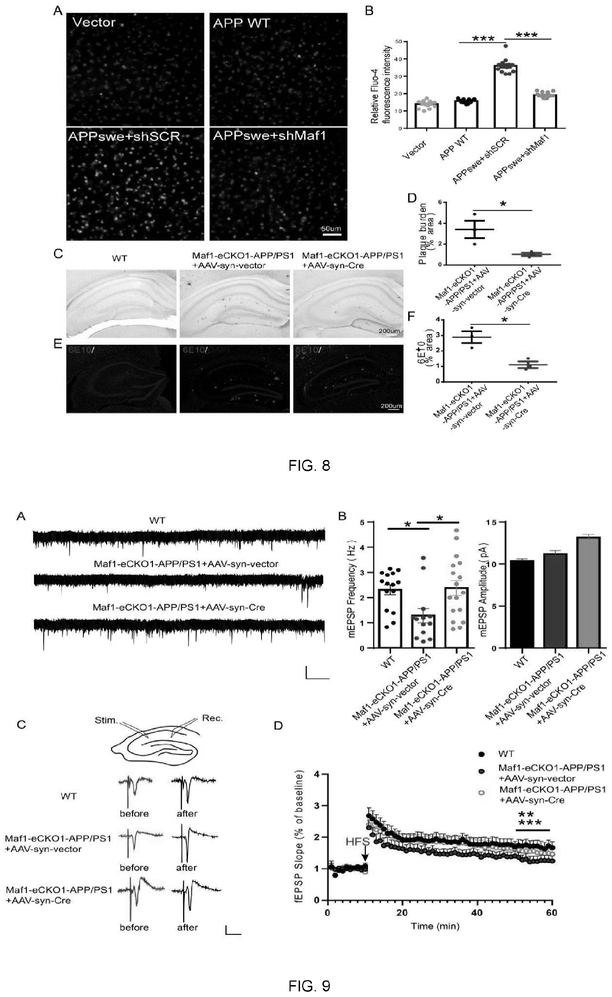

FIG. 8 shows that the knockout of Maf1 affects calcium homeostasis and amyloid plaque deposition in hippocampal neurons of AD.
   (A) Primary hippocampal neurons cultured *in vitro* after infection with APP, shSCR and shMaf1 lentiviruses. The green fluorescence under the fluorescence microscope represents the intensity of Fura-4/AM. (B) A statistical quantification chart of fluorescence intensity obtained from group 4. (C-D) Immunohistochemical and statistical results of Aβ plaques in 6-month-old Maf1-eCKO1-APP/PS1 mice. (E-F) Immunofluorescence and statistical results of Aβ plaques in 6-month-old Maf1-eCKO1-APP/PS1. *p<0.05; **p<0.01; ***p<0.001.
FIG. 9 shows that the conditional knockout of Maf1 is able to rescue synaptic dysfunction in AD mice. (A) mEPSC of hippocampal CA1 neurons in WT and Maf1-eCKO1-APP/PS1 mice injected with AAV-syn-Cre-GFP and AAV-syn-GFP vectors is recorded according to the procedure of acute brain slice electrophysiological experiments. (B) The average mEPSC amplitude is shown on the right, and the average mEPSC amplitude is shown on the left. Calibration: 15 Pa, 1 s. WT, n = 15 neurons from 4 mice; AAV-syn-vector, n = 12 neurons from 4 mice; AAV-syn-Cre, n = 12 neurons from 4 mice. (C) The figure above shows the fEPSP record of the CA3-CA1 pathway. A stimulating electrode is placed in the Schaffer collateral region of CA3, and a recording pipette is placed in the stratum radiatum of CA1. The electrode is stimulated and the pipette is recorded. In LTP recordings, the records at the baseline and the last 10 min represent fEPSP recordings. Calibration: 0.5 mV, 10 ms. (D) Quantitative analysis of the average fEPSP slope during the last 10 min of LTP in 4 mice: WT, n = 9 neurons; AAV-syn-vector, n = 9 neurons from 4 mice; AAV-syn-Cre, n = 11 neurons from 4 mice. The data are presented as mean±SEM. *p < 0.05; **p < 0.01; ***p < 0.001.

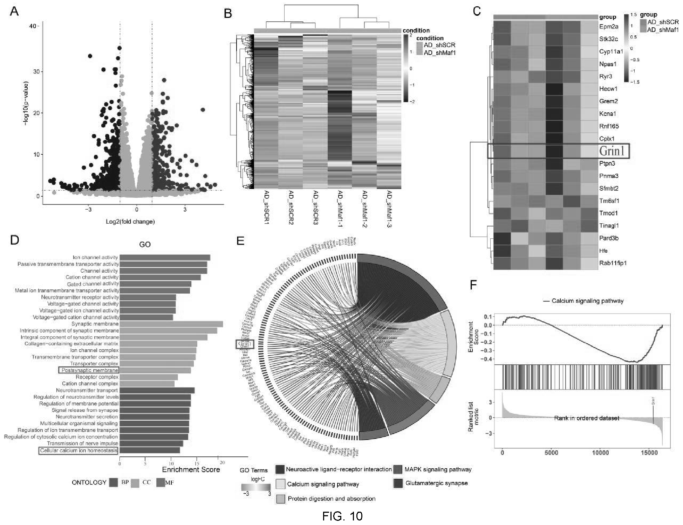

FIG. 10 shows that RNAseq analysis indicates that Maf1 regulates the downstream gene Grin1, which is involved in calcium signaling pathways and synaptic functions.
   (A) RNAseq sequencing is performed to determine changes in neuronal gene expression profiles after Maf1 deletion in AD. The volcano plot shows 884 differentially expressed genes, wherein 320 genes are up-regulated (red scatter) and 564 genes are down-regulated (blue scatter). (B) The heatmap of differentially expressed genes shows differences in gene expression profiles between two groups. (C) GO enrichment analysis is performed for different genes, and the top 30 entries are shown in the figure. (D) The differential heatmap shows that the Grin1 gene is significantly downregulated after Maf1 deletion. (E) KEGG pathway enrichment map of the top 5 differentially expressed genes. (E) Gene set enrichment analysis (GSEA) shows that the calcium ion signaling pathway is associated with Grin1.

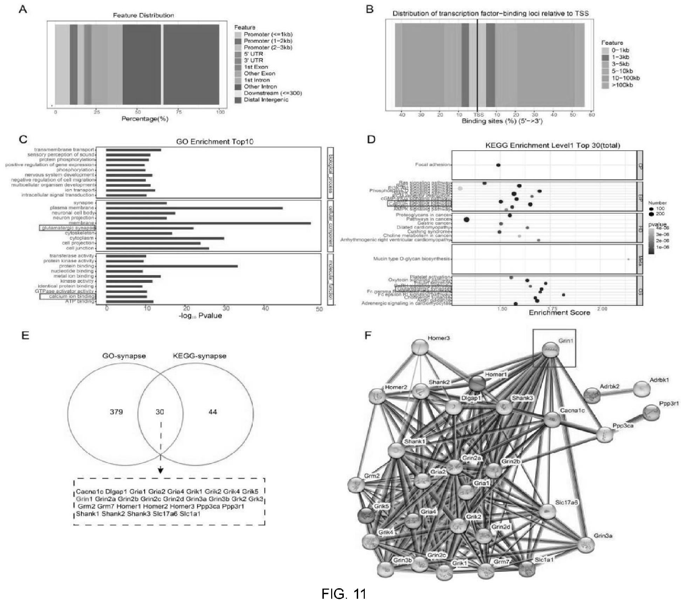

FIG. 11 and FIG. 6 show that CUT&Tag sequencing demonstrates protein-DNA interactions between the Maf1 and Grin1 genes. (A) An annotation map of Maf1 binding peaks in the gene functional element region. (B) A distribution plot of the distance between Maf1 binding peaks and gene transcription start site (TSS). (C) The top 10 entries of GO enrichment analysis of the binding site genes are related to the binding of synapses and calcium ions. (D) The top 30 entries of KEGG enrichment analysis for binding sites show that the calcium signaling pathway is closely related to synaptic secretion of glutamine. (E) Schematic diagram of the co-intersection genes from GO-synapse and KEGG-synapse enriched genes. (F) GO and KEGG protein interaction network analysis shows that Grin1 is located at the core.

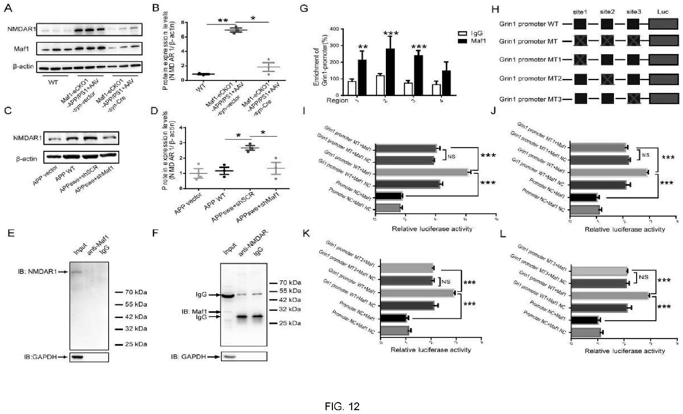

FIG. 12 shows that Maf1 regulates the activity of a Grin1 promoter and promotes the expression of NMDAR1.
   (A) Protein levels of NMDAR1 in mouse hippocampus; (B) Statistical quantification of the in vivo Western blot results. (C) Changes in the content of NMDAR1 protein in primary neurons; (D) Statistical quantification of the in vitro Western blotting results. (E-F) Immunoprecipitation of Maf1 and NMDAR1 in hippocampal neurons. Maf1 does not directly bind to NMDAR1. (G) 4 sequences are designed according to the Grin1 promoter for primer synthesis. The ChIP-quantitative PCR results show that four sequences in fragments 1, 2 and 3 are enriched in IP-Maf1. (H) A schematic diagram of constructing wild-type Grin1 promoter luciferase (Luc) and mutant Grin1 promoter luciferase plasmids. (I) The Grin1 wild-type and mutant-type (MT) promoter plasmids are co-transfected with pcDNA-NC and pcDNA-Maf1 into HEK293T cells. Detecting luciferase activity using a dual-luciferase reporter gene assay. (J-L) Grin1 wild-type and mutant-type promoter plasmids (MT1, MT2, MT3) were co-transfected with pcDNA-NC and pcDNA-Maf1 into HEK293T cells.

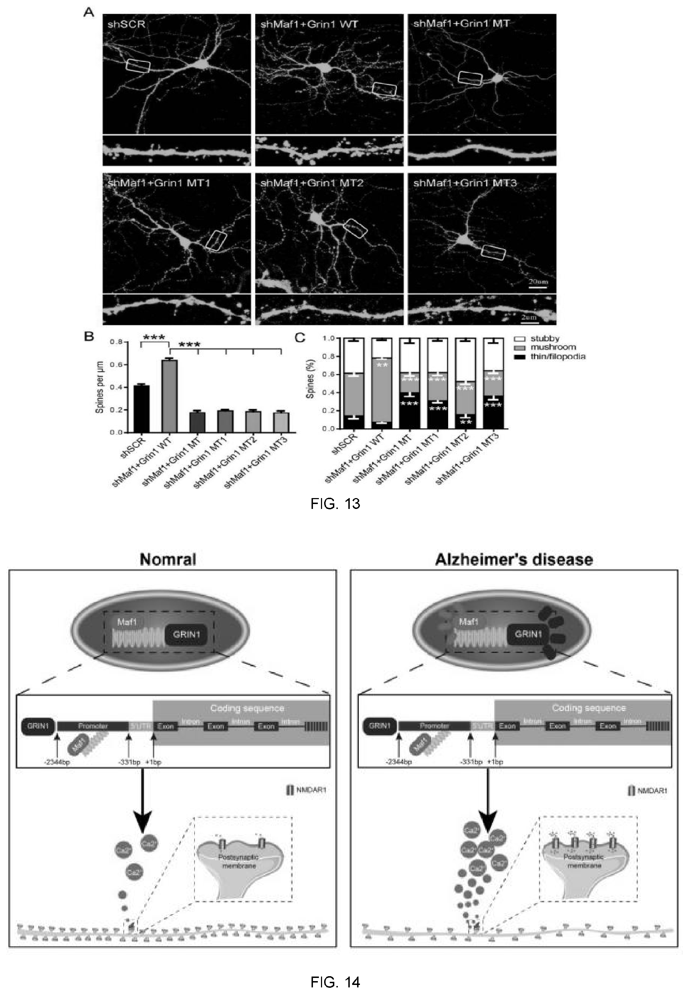

FIG. 13 shows that the mutation of a Grin1 promoter attenuates the morphological effects of Maf1 knockdown.
   (A) Representative GFP fluorescence images under a fluorescence microscope after transfection of primary hippocampal neurons with shSCR, shMaf1 as well as Grin1WT and MT plasmids. The following is a partial magnification of the white box. Scale: above, 20 µm; below, 2 µm. (B) Statistical analysis of the density of dendritic spines of in cultured neurons. (C) A statistical chart of the percentage classification of dendritic spines in cultured neurons. n = 7-20 neurons per group. The data are presented as mean±SEM. *p < 0.05; **p < 0.01; ***p < 0.001.
   FIG. 8 shows a mechanism that the Maf1-NMDAR1 signaling pathway regulates neuronal calcium homeostasis and participates in synaptic remodeling in Alzheimer's disease. In AD, the expression of the transcriptional regulator Maf1 increases, wherein Maf1 further regulates calcium homeostasis by binding to the promoter region of Grin1, and alters the morphological structure and quantity of dendritic spines, thereby affecting synaptic function and consequently regulating the expression of NMDAR1. Synaptic remodeling is ultimately regulated, thereby affecting learning and memory functions.
FIG. 15 shows the establishment and detection of a Maf1 protein-test substance interaction assay model.
FIG. 16 shows the results and trends of changes in blood drug concentration in mice.
FIG. 17 shows the detection of compound content changes in mouse brain tissue and comparison of blood/brain tissue content.
FIG. 18 shows an IC50 curve of a compound of Formula I in a 293T cell line.
FIG. 19 shows an IC50 curve of a compound of Formula I in primary neurons.
FIG. 20 shows an IC50 curve of a compound of Formula II in a cell line and primary neurons.
FIG. 21 shows that compound intervention is able to improve dendritic spine density of neurons of an in vitro AD model.
FIG. 22 shows that compound intervention is able to improve the learning and memory abilities of an AD animal model.
FIG. 23 shows that compound intervention is able to improve the dendritic spine density of neurons in an AD animal model.
FIG. 24 shows changes in hippocampal tissue gene transcriptomics after compound intervention in AD.

### Detailed Description of the Embodiments

The specific embodiments of the present disclosure are further described below. It is to be noted that the description of these embodiments is used to help understand the present disclosure, but does not constitute a limitation to the present disclosure. In addition, the technical features involved in the embodiments described below can be combined with each other as long as they do not conflict with each other.

The "diagnosis of Alzheimer's disease" described herein comprises both determining whether a subject has already suffered from Alzheimer's disease and determining whether the subject is at risk of suffering from Alzheimer's disease.

The term "Aβ" described herein is β-amyloid protein.

The term "NFTs" described herein refers to neurofibrillary tangles.

The term "NMDA" described herein refers to the NMDA receptor, N-methyl-D-aspartate receptor, which is an ionotropic glutamate receptor subtype. As a key excitatory ionotropic glutamate receptor, it is crucial in the formation of the nervous system and synaptic plasticity.

The term "CaN" as used herein refers to Ca²⁺-calmodulin-dependent phosphatase.

The term "CaMKII" as used herein refers to calcium/calmodulin-dependent protein kinase II.

The term "LTP" as used herein refers to long-term potentiation.

The term "LTD" as used herein refers to long-term depression.

The term "mEPSC" as used herein refers to miniature excitatory postsynaptic potential.

The term "fEPSPs" as used herein refers to field excitatory postsynaptic potentials.

The term "HFS" as used herein refers to high-frequency stimulation.

The term "ACSF" as used herein refers to artificial cerebrospinal fluid.

The term "MWM" as used herein refers to the MWM test: Morris water maze test.

The experimental methods in the following examples, unless otherwise specified, are all conventional methods, and the experimental materials used in the following examples, unless otherwise specified, can be purchased through conventional commercial channels.

### Examples

### 1. Animal

APP/PS1 transgenic mice with the C57BL/6J genetic background were derived from Shanghai Model Organisms. Maf1-eCKO1 transgenic mice were constructed by Shanghai Model Organisms. The flox was recombined via homologous modification of the Maf1 gene fertilized egg. Based on the principle of homologous recombination, CRISPR/Cas9-mediated gene editing was utilized on mice with the C57BL/6J background. By tissue-specific infection via local injection of AAV-Cre virus combined with a neuron-specific promoter driving the Cre gene, stronger tissue-specific and neuron-specific gene recombination can be achieved. The Maf1-eCKO1 transgenic mice were further crossed with APP/PS1 mice to obtain Maf1-eCKO1-APP/PS1 transgenic mice. ICR mice were purchased from Shanghai Jiesijie Laboratory. The animals were housed in a specific pathogen-free environment (23±2°C, 45±5% humidity, 12 h light/dark cycle) with free access to pellet feed and water.

### 2. Generation of plasmid and viral constructs

Three shRNA target sequences and one scramble shRNA sequence were designed, and then primers were synthesized based on the Maf1 gene. The primer sequences are as follows:
shRNA1: TTGGAGAACTCCAGCTTTGAGGCCATCAA;
shRNA2: TCTGCTTAGCTGAGTG TGACATCTACAGC;
shRNA3: CCTCAATGAGTCCTTCCGGCCAGACTATG;
scramble shRNA (shSCR): GCACTACCAGAGCTAACTCAGATAGTACT.

The following single-stranded primers were annealed into double-stranded oligo sequences and linked to a double-enzyme-digested linearized RNA interference vector. The transformants were verified for correctness by sequencing, and high-purity plasmids were extracted.

The maf1 shRNA plasmid was constructed using the pGFP-CMV-ShLenti vector, and further packaged into lentiviruses. The Maf1-interfering adeno-associated virus was constructed using the pAAV-hSyn-EGFP-3xFLAG-WPRE vector. The Cre-interfering adeno-associated virus was constructed using the pAAV-hSyn-EGFP-WPRE vector. The APP695swe (K595N/M596L) virus was expressed by the pSLenti-CMV vector. Both the lentivirus and the adeno-associated virus were commissioned by Obio Technology (Shanghai), and stored at -80°C.

### 3. Cell culture and transfection

A primary hippocampal culture was prepared from the brains of embryonic mice aged 18 days (Xu, N.J. and M. Henkemeyer, *Ephrin-B3 reverse signaling through Grb4 and cytoskeletal regulators mediates axon pruning.* Nat Neurosci, 2009. **12**(3): p. 268-76.). Neurons isolated from hippocampal tissue were inoculated with polylysine and cultured in a neurobasal medium containing a B27 supplement and GlutaMAX. For lentivirus-infected neurons, on day 6 in vitro, concentrated lentiviruses were added to the medium and the cells were transfected. The transfection efficiency was verified by fluorescence and protein levels after cell lysis. APPswe viruses were transfected for 6 days in vitro (DIV), and then shRNA plasmids were transfected with calcium phosphate for 2 days, with 10 days in total, so as to verify the regulatory effect of Maf1 on neuronal dendritic growth. When two plasmids were simultaneously transfected, the plasmid carrying GFP was mixed with another plasmid at a ratio of 1:3.

### 4. Real-time quantitative polymerase chain reaction (PCR)

The total RNA of primary hippocampal cultures was extracted using Trizol reagent (Invitrogen, Carlsbad, CA, USA). Then, RNA was reversely transcribed into cDNA using a Hifair^{®} RT kit (Yeasen, Shanghai, China). Real-time qPCR was performed using Hieff UNICON^{®} qPCR SYBR Green Master Mix (Yeasen) on a 7500 Real-Time PCR System (Applied Biosystems, Foster City, CA, USA).

### 5. Western blotting

The hippocampus of mice was dissected, homogenized, and dissolved in a lysis buffer at 4°C for 30 min. The lysate was centrifuged at 12,000 g for 20 min at 4°C to remove insoluble precipitates, and the protein concentration was estimated using a bicinchoninic acid (BCA) protein assay kit (Thermo Scientific Rockford, IL, USA). Then, the protein was boiled in a sodium dodecyl sulfate (SDS) loading buffer at 95°C for 10 min for denaturing. The protein was run on a 10% polyacrylamide gel, and then transferred to a nitrocellulose membrane. At room temperature, the membrane was blocked in Tris buffered saline Tween-20 (TBST) (150 mM NaCl, 10 mM Tris, 0.1% Tween 20, pH 7.6) containing 5% BSA for 1 h. The primary antibody was diluted with a blocking buffer, and then incubated at 4°C overnight. After washing with TBST, the blot was incubated with an HRP-conjugated secondary antibody at room temperature for 1 h. After washing again, the blot was exposed to an enhanced chemiluminescence substrate. The relative density of the exposed membrane was quantitatively analyzed using ImageJ software. For the primary antibody, we used rabbit anti-Maf1 (1:500, Abcam, catalog ab230499), rabbit anti-β-actin (1:1000, Cell Signaling Technology, catalog 8457S), mouse anti-GAPDH (1:3000, Invitrogen, catalog MA5-15738-D680), rabbit anti-NMDAR1 (1:1000, Abcam, catalog ab109182), mouse anti-NMDAR1 (1:1000, Abcam, catalog ab134308), rabbit anti-APP (6E10) (1:1000, Novusbio, catalog NBP2-62566) and immunoprecipitation-rabbit IgG (1:1000, Abcam, catalog ab172730).

### 6. Immunohistochemistry and Immunofluorescence

Primary hippocampal neurons were fixed with PBS containing 4% paraformaldehyde (PFA) for 30 min and stored at 4°C. The mouse was perfused, and the brain was fixed with a 4% PFA solution in PBS at 4°C overnight. 30 µm coronal brain sections were generated using a VT1000 vibrating microtome (Leica Biosystems, Wetzlar, Germany). For immunohistochemistry (IHC), the activity of an endogenous peroxidase was blocked using 3% H₂O₂ at room temperature for 10 min, and then the sections were blocked with a blocking solution (PBS solution containing 0.03% Triton X-100 (MilliporeSigma) and 10% donkey serum (Invitrogen)) at room temperature for 10 min. The sections were then incubated with the primary antibody in an antibody solution (PBS containing 0.03% Triton X-100 and 2% donkey serum) at 4°C overnight. On the next day, the sections were incubated with an enzyme-conjugated anti-rabbit antibody, and stained using the DAB kit (Vector Labs, Carlsbad, CA, USA). For immunofluorescence (IF), the sections were blocked with a blocking buffer for 30 min and incubated with the primary antibody, and the nucleus was stained with DAPI in the antibody solution overnight. The sections were rinsed in PBS for 10 min and then incubated with an Alexa Fluor secondary antibody for 2 h. The sections were rinsed in PBS for 10 min, then mounted with a mounting medium, and imaged. The following antibodies were used: rabbit anti-Maf1 (1:150, Abcam, catalog ab230499), chicken anti-Map2 (1:10000, Abcam, catalog ab5392), Alexa Fluor secondary antibodies (Alexa Fluor 488, Alexa Fluor 555, Alexa Fluor 647; 1:500, Thermo Fisher).

### 7. Image analysis and quantification

High-resolution images for double immunofluorescence experiments were obtained using confocal microscopy (Leica SP8). A z-series of 7-12 images were captured at a resolution of 1024×1024 pixels, with a depth interval of 0.5 µm-1 µm, and averaged each frame twice. By using maximum intensity projection, the synthetic stack was flattened into a single image (Jaworski, J., et al., Control of dendritic arborization by the phosphoinositide-3'-kinase-Akt-mammalian target of rapamycin pathway. J Neurosci, 2005. 25(49): p. 11300-12.). The fluorescence intensity and cellular localization of the target protein were measured by using a ×20 objective lens. To analyze dendritic spines, neurons were imaged using a ×63 objective and 2× zoom with a pixel resolution of 1024 × 1024. The spine shapes were further quantitatively determined, and neuronal spine shapes were classified to evaluate GFP-positive cells. Image J was used.

### 8. RNA sequencing and data analysis for quantification

Total RNA was extracted by using a mirVana miRNA Isolation Kit (Ambion). The RNA integrity was evaluated using an Agilent 2100 bioanalyzer (Agilent Technologies, Santa Clara, CA, USA). Subsequent analysis was performed on samples with RNA integrity numbers (RIN)≥7. Libraries were constructed using a TruSeq Stranded mRNA LT Sample Prep Kit (Illumina, San Diego, CA, USA). Then these libraries were sequenced on an Illumina sequencing platform (HiSeqTM 2500 or Illumina HiSeq XTen) to generate 125 bp/150 bp paired-end reads. Transcriptome sequencing and analysis were performed by OE biotech Co., Ltd. (Shanghai, China). The hippocampal and brain tissues isolated after anesthesia were isolated for cutting and labeling analysis. The lysis buffer was added to the collected tissues to dissociate tissue masses, so as to promote rapid release of nuclei. ConA magnetic beads resuspended in Binding buffer were added. The target protein was bound to the primary antibody and incubated with the secondary antibody. Next, the ChiTag transposon was used to bind the antibody, and then the ChiTag transposon was activated to fragment the target DNA. Finally, DNA was extracted and subjected to PCR, and then the product obtained after DNA purification is subjected to computer sequencing. Shanghai Ouyi Biomedical Technology Co., Ltd. provided assistance in sequencing and data analysis.

### 9. Co-Immunoprecipitation (CoIP) Assay

The Thermo Scientific IP lysis buffer (Catalog No.: 87787) was used. 1 µg of Maf1 antibody or NMDAR1 antibody was added to the protein extract. The mixture reacted overnight at 4°C, and then the reaction product was incubated with protein G beads for 3 h at 4°C. The product obtained after incubation was subjected to immunoblotting assay and detected with a specified antibody.

### 10. Chromatin Immunoprecipitation (ChIP)

The ChIP assay was performed using the Pierce^{™} Agarose ChIP Kit (Thermo Fisher Scientific). Chromatin was extracted, and DNA was cut into fragments of 0.2 kb-1 kb. Primary hippocampal neurons were crosslinked with 1% formaldehyde, and chromatin was immunoprecipitated using the antibody Maf1 (Abcam). IgG was used as a negative control. DNA was resuspended in 50 µL of TE buffer and subjected to PCR amplification. The PCR product was determined on a 1.5% agarose gel. DNA (TransGen Biotech, Catalog No.: EP101) was purified, followed by further analysis through qPCR using a primers specific for Grin1 promoter.

The ChIP-PCR primers used in the present study are as follows:
Grin1-promoter-F1: GCCCAAGAAGAGCCCAAGAA,
R1: CCAGGGAAGATTTGTCACGC;
Grin1-promoter-F2: GACAGGGGTCTTAGACAGTGC,
R2: ACCCACACGTTCACACTCC;
Grin1-promoter -F3: CTTTACATGGCAGGAGGGGG,
R3: CATCTTGCTCCCAGCAGTCA;
Grin1-promoter-F4: CGGATCCAAGGTGGTGTT,
R4: TCTAGAGGGTGAGATAGGATTGCT.

### 11. Dual-luciferase reporter gene assay

Interaction between Maf1 and a Grin1 promoter region. The plasmid vector was the pGL3 promoter vector. The Maf1 overexpression plasmid, the wild-type Grin1 promoter luciferase (Luc) and the mutant Grin1 promoter luciferase were constructed by Genomeditech Co. Ltd. The cells were transfected with WT or MT plasmids and Maf1 or a negative control using Lipofectamine 2000 (Invitrogen, Thermo Fisher Scientific, Inc.). After transfection for 48 h, the relative luciferase activity was determined using a dual-luciferase reporter assay kit (Promega, Madison, WI, USA). The firefly luciferase activity was normalized to Renilla luciferase activity. The experiment was performed in triplicate. Mutations on the Grin1 promoter were constructed at -1774 to -951 (MT), -1774 to -1697 (MT1), -1308 to -1217 (MT2), and -1039 to -951 (MT3).

### 12. Calcium imaging assay

Primary hippocampal neurons were treated with APP_{Swe} lentiviruses and shMAF1 lentiviruses in DIV8. The calcium imaging assay was performed in approximately DIV14-DIV16. Neurons with a final concentration of 2 µmol/ml Fura-4 AM (Beyotime, Cat. No.: S1061M) were incubated at 37°C for 30 min. After rinsing with physiological saline, the cells were taken out at 340 nm and observed at 510 nm using an inverted fluorescence microscope.

### 13. Stereotactic injection

The mice were anesthetized using a 1% sodium pentobarbital solution (50 mg/kg, intraperitoneal). A hole was drilled in the hippocampal CA1 region (anterior/posterior = ±1.25 mm, medial/lateral = ±1.7 mm, dorsal/ventral = ±1.6 mm). The AAV2/9-syn-shSCR-GFP, AAV2/9-syn-shMaf1-GFP, AAV-syn-GFP or AAV-syn-Cre-GFP vector (10¹² IU/mL, 2 µL) was bilaterally microinfused into the hippocampus via a cannula connected to a Hamilton (Reno, NV) microsyringe. The infusion rate was 0.2 µL/min, and the cannula was left in place for 10 min after completion of the infusion.

### 14. Morris water maze

Learning and memory functions were evaluated 1 month after AAV2/9 injection. The Morris water maze was a white circular pool (diameter 120 cm) filled with water and dyed white with milk. The platform (with a diameter of 6 cm and a height of 30 cm) was placed in one of the quadrants of the water tank. The pool was filled with water at 20±1°C, and the platform was hidden 1 cm below the surface. The mice were placed in the same room as the water maze one day before the assay to adapt to the environment. During the collection phase, the mice were gently placed into the water from the inner wall of the pool in the non-target quadrant. At the same time, the recording of the assay started, with a total recording time of 60 s. If the mice found the platform and stayed for 3 s or longer, it was determined that the mice find the platform, and the recording was automatically stopped. For mice that failed to find the platform within 60 s, the latency was recorded as 60 s. The mice should be guided to stay on the platform for 20 s. The latency and travel distance of the mice to find the platform were recorded. Space exploration phase: on day 6, the circular platform in the target quadrant was taken out, and the farthest straight-line distance from the platform was selected as the water entry point for the mouse. The mice were allowed to swim in water for 60 s, and the parameters including the number of platform crossings, total travel distance, and the percentage of time spent in each quadrant were recorded. By using the ANY-maze automatic video tracking system (MED Associates, Georgia, VT, United States), all activities of the mice were recorded via a camera video, and the collected results were calculated for statistical analysis to measure the latency to reach the platform position, the percentage of time spent in the target quadrant, and the number of crossings over the platform.

### 15. Golgi apparatus staining

The mice were anesthetized using a 1% pentobarbital sodium solution (50 mg/kg, intraperitoneal). The brain tissue was immersed in a mordant solution for 3 days, then soaked in a 1.5% AgNO₃ solution at room temperature in a dark environment for 5 days, dehydrated in a gradient ethanol series (70%, 2 h; 80%, 2 h; 90%, overnight; 95%, 90 min×2; and 100%, 40 min×2), cleared in xylene (20 min×2), embedded in paraffin, and serially sectioned into 60 µm slices. The digital images with 1000× magnification were used, and 15 or more fully impregnated neurons that were not obscured by adjacent neurons were randomly selected from the sections of each group of mice for analysis. The dendritic spine density (number/µm) was calculated using ImageJ.

### 16. Electrophysiological recording of brain slices

The brain was quickly dissected and placed in a solution containing: 125 mM NaCl, 2.5 mM KCl, 2 mM CaCl₂, 1 mM MgCl₂, 25 mM NaHCO₃, 1.25 mM NaH₂PO₄, and 12.5 mM glucose. Coronal brain slices (300 µm thick) were prepared using a vibratome, incubated in ACSF containing 95% O₂ and 5% CO₂ at 31°C for 1 h, and then stored at room temperature (22-25°C). In the presence of 100 µM microtoxin and 1 µM tetrodotoxin, a miniature excitatory postsynaptic potential (mEPSC) of -70 mV was recorded. To record long-term potentiation (LTP), extracellular field excitatory postsynaptic potentials (FESPs) were evoked in the Schaffer collateral pathway and the CA1 region was recorded. LTP was induced by high-frequency stimulation (HFS), which consisted of 1-sec 100 Hz sequences at an intensity of 70-80% of each sequence that eliciting peak fEPSPs. The data were analyzed in pCLAMP 10.6 (Molecular Devices), with an average of 3 cells per plate and 2-3 cells were recorded per mouse.

### 17. Statistical analysis

All statistical analyses were performed using the program GraphPad Prism 6.01. The data were presented as mean±SEM of at least 3 biological replicates in the experiment. One-way ANOVA or two-way ANOVA was used, followed by Dunnett's test or t-test. Non-normal distributions were analyzed using the Kruskal-Wallis test. When p < 0.05, it was determined that the difference between groups was statistically significant. An asterisk indicates statistical significance: *p < 0.05; **p < 0.01; ***p < 0.001.

### Example 1 Expression of Maf1 in hippocampus of AD patients and APP/PS1 mice

The memory decline and intellectual loss in AD are directly related to the morphology and function of neurons. Maf1, as a transcription factor, may regulate the presence of other genes in the pathogenesis of AD. Therefore, genomic research was conducted on the human brain GEO dataset GSE5281, and analysis was performed using R software version 4.0.5 and the Limma package. In doing so, all differential genes between AD and normal hippocampal tissues could be found.

The experimental results demonstrated that compared with the corresponding non-dementia control groups (n=13 and n=10, respectively), the expression of Maf1 in the human brain dataset of AD patients was elevated; that is, the expression level of Maf1 was significantly increased in AD (FIGs. 1A, 1B, and 1C; P = 0.007256926).

During the in vitro culture, primary hippocampal neurons were transfected with APPswe viruses. The results showed that compared with the Vector and APP WT viruses, the Maf1 protein level in primary hippocampal neurons transfected with the APPswe viruses was significantly increased (FIGs. 1D and 1E). Meanwhile, proteins were extracted from the hippocampus of 6-month-old APP/PS1 mice. It was found that the level of Maf1 protein was also significantly increased (FIGs. 1F and 1G). In addition, it was confirmed by immunohistochemistry and immunofluorescence that the expression of Maf1 protein in AD was increased (FIGs. 1H-1K). Therefore, these data indicated that the expression level of Maf1 was abnormally elevated in AD.

To further verify whether the results hereinabove exist in neurons of AD models and AD transgenic mice, and to explore the expression of Maf1 in hippocampal neurons, primary hippocampal neurons extracted from fetal rat hippocampus were used as an in vitro model. First, NeuN staining was used to verify the purity of the primary neurons extracted in vitro. The expression of Maf1 in hippocampal neurons in vivo and in vitro was explored using immunofluorescence techniques.

The experimental results are shown in FIG. 2A. In the figure, NeuN expression was found in more than 90% of the cells, and the extracted cells were proven to be relatively pure primary neurons.

The experimental results are shown in FIG. 2B. In the figure, Maf1 and MAP2 (a neuronal marker) were co-labeled in brain slices and primary neurons in vitro, indicating that Maf1 was expressed in both the soma and dendrites of hippocampal neurons.

### Example 2 Conditional knockout of Maf1 in hippocampal neurons improves learning and memory functions

Maf1 conditional knockout mice (Maf1-eCKO1) were generated using the CRISPR-Cas9 system to study the effect of Maf1 deletion on hippocampal neurons in the AD brain. Guide RNA targeting specific exons were designed to construct Maf1-eCKO1 mice, and the transgenic mice were crossed with APP/PS1 mice to obtain Maf1-eCKO1-APP/PS1 mice (FIGs. 3A-3B). Genotyping was performed from mouse tail tips. PCR confirmed homozygous Maf1 gene flox and APP/PS1 mice capable of performing conditional knockout (FIG. 3C). At 5 months, the AAV-hsyn-Cre-GFP or AAV-hsyn-GFP vector was injected into the hippocampal CA1 region of Maf1-eCKO1-APP/PS1 mice by stereotactic microinjection (FIGs. 3D and 3E). One month after virus injection, the mice were subjected to an MWM learning and acquisition experiment for 5 days, and a probe test was performed on day 6 to determine the ability of the mice to locate the quadrant that previously contained the hidden platform (FIG. 3E). After injection of AAV-hsyn-Cre-GFP into the hippocampal region, immunofluorescence confirmed the loss of Maf1 protein, and WB analysis was performed on the mouse hippocampus (FIGs. 3F and 3G).

The experimental results showed that by comparing the control with the Maf1-eCKO1-APP/PS1 mice injected with the virus, the Maf1 band of the Maf1-eCKO1-APP/PS1 mice injected with AAV-hsyn-Cre-GFP was significantly reduced (as shown in FIG. 3F).

After the effectiveness of the conditional knockout was verified, the mice were subjected to Morris water maze behavioral test to determine the effect of hippocampal Maf1 expression on learning and memory behaviors. During the acquisition test, the Maf1-eCKO1-APP/PS1 + AAV-hsyn-Cre-GFP mice exhibited significantly shorter mean escape latencies on training days 2, 3, 4, and 5 compared with the Maf1-eCKO1-APP/PS1 + AAV-hsyn-GFP vector mice; moreover, conditional knockout of Maf1 resulted in reduced latency on day 5 in the Maf1-eCKO1-APP/PS1 + AAV-hsyn-GFP mice compared to the Maf1-eCKO1-APP/PS1 + AAV-hsyn-GFP vector mice (FIGs. 4A and B).

From the probe test, the percentage of time spent in the target quadrant was first analyzed. It was found that WT and Maf1-eCKO1-APP/PS1 + AAV-hsyn-Cre-GFP mice primarily swam within or near the target quadrant, whereas Maf1-eCKO1-APP/PS1 + AAV-hsyn-GFP vector mice swam less frequently near the target quadrant (FIG. 4C). Compared with the Maf1-eCKO1-APP/PS1 + AAV-hsyn-GFP vector mice, the Maf1-eCKO1-APP/PS1 + AAV-hsyn-Cre-GFP mice spent more time in the target quadrant (FIG. 4D). In addition, the platform crossing times of Maf1-eCKO1-APP/PS1 + AAV-hsyn-Cre-GFP mice were significantly higher than those of Maf1-eCKO1-APP/PS1 + AAV-hsyn-GFP vector mice (FIG. 4E).

In summary, these results indicated that Maf1 was of great significance to the cognitive function of AD mice.

### Example 3 Effect of Maf1 knockdown on neural synapses

Studies have shown that synaptic loss and dysfunction will more directly lead to the decline of the cognitive function in the brains of AD patients. This manifests as a reduction in the number of mushroom dendritic spines, a decline in learning and memory, etc. These changes in neuronal morphology and basic functions lead to the loss of cognitive function in AD patients. It is worth noting that the structure, quantity and function of synapses are crucial for maintaining brain function.

The spine density was detected using Golgi-Cox staining (FIG. 5A) to determine whether the behavioral recovery induced by Maf1 knockout was associated with structural changes in dendritic spine density in vivo. Compared with the Maf1-eCKO1-APP/PS1 + AAV-hsyn-GFP vector mice, the spine density of Maf1-eCKO1-APP/PS1 + AAV-hsyn-Cre-GFP mice was increased by 18% (FIGs. 5B and 5C); the density of mushroom-like spines was also increased by approximately 10%, while the density of thin/filopodia-like spines was decreased by approximately 17% (FIG. 5D). It was suggested that the reduction of Maf1 was able to significantly restore dendritic spine damage in AD hippocampal neurons.

Therefore, primary neurons were extracted, transfected with APP lentiviruses, and then transfected with shSCR and shMaf1 plasmids to verify this phenomenon in an in vitro AD model (FIG. 5E). Consistent with the in vivo studies, the knockout of Maf1 increased the density of mushroom spines and decreased the density of filopodia/thin protrusions in vitro (FIGs. 5F and 5G).

In general, the knockdown of endogenous Maf1 expression in hippocampal neurons can promote the growth and development of neuronal dendritic spines under AD pathological conditions and increase the density of dendritic spines. Moreover, the type of dendritic spines is mainly the mushroom type. It can be seen that Maf1 plays a crucial role in the progression of AD by regulating synapses through modulating dendritic spines.

To verify this phenomenon in vitro, three shRNA sequences were designed, including a scrambled shRNA with nonspecific sequence interference as a control sequence and a Maf1 overexpression plasmid with a Flag tag (Maf1-OE).

The experimental results showed that 72 h after lentivirus transfection of 293T cells, a high GFP green fluorescence intensity was observed under a fluorescence microscope (FIG. 6A); compared with shSCR-transfected neurons, the ShRNAs targeting Maf1 effectively reduced the endogenous protein level, wherein shMaf1-2 exhibited the best effect (FIG. 6B). In addition, a significant increase in the expression of Maf1 was detected in the WB experiment, and the presence of the marker protein confirmed the successful overexpression of Maf1 (FIG. 6C).

Mature primary hippocampal neurons were further transfected in vitro using ShSCR, shMaf1, and Maf1-OE lentiviruses. It was observed that both shMaf1-2 and shMaf1-3 lentiviruses were able to effectively reduce the expression level of Maf1 in hippocampal neurons (FIG. 6D). After transfection with the Maf1-OE lentiviruses, the protein level of Maf1 was also significantly increased (FIG. 6E). All subsequent viral experiments were performed using shMaf1-3, with shMaf1 as the representative.

It was found that Maf1 was highly expressed in neuronal dendrites and dendritic spines (FIG. 6F).

AAV-syn-shSCR-GFP and AAV2/9-syn-shMaf1-GFP were injected into the hippocampus of AD mice for research. The effect of Maf1 knockdown in the mouse hippocampus was confirmed by immunofluorescence and WB analysis (FIGs. 7A and 7B).

Consistent with the phenomenon observed in Maf1-eCKO1-APP/PS1, the knockout of Maf1 can affect the number and morphology of neuronal dendritic spines (FIGs. 7C-F) and improve the learning and memory dysfunction in mice (FIGs. 7G-I).

### Example 4 Effect of Maf1 on calcium homeostasis and amyloid plaque deposition in AD hippocampal neurons

In neural activities, a plurality of biological effects, such as synaptic transmission of nerve cells, neurotransmitter release, cell morphology and growth, and activation of various enzymes, are related to calcium signaling pathways. Calcium signaling in dendritic spines plays an important role in controlling synaptic plasticity. Previous studies have also demonstrated that calcium imbalance may be an important cause of AD onset, the Ca²⁺ level in the endoplasmic reticulum of AD and aging neurons increases, leading to subsequent compensatory changes and defects in neuronal Ca²⁺ signal transduction. In view of increasing evidence showing that Ca²⁺ overload is able to block LTP, leading to synaptic loss and neurodegeneration, it was subsequently determined whether Maf1 affects neuronal morphology and function by regulating intracellular Ca²⁺ signaling. Primary hippocampal neurons were extracted, transfected with APP lentiviruses, and then transfected with shSCR and shMaf1 plasmids. Then, total Ca²⁺ influx was estimated using the Fura-4/AM calcium imaging method.

Under AD conditions, it was found that the Ca²⁺ influx in primary hippocampal neurons was significantly increased; moreover, the knockout of Maf1 reduced the level of Ca²⁺ influx (FIGs. 8A and 8B). Therefore, our findings suggest that Maf1 can regulate calcium concentration in primary hippocampal neurons.

To further determine whether the increased expression level of Maf1 is related to AD pathology, we performed Aβ immunohistochemistry and immunofluorescence staining. We found that compared with Maf1-eCKO1-APP/PS1 + AAV-hsyn-GFP vector mice, Maf1-eCKO1-APP/PS1 + AAV-hsyn-Cre-GFP mice showed a significant reduction in hippocampal Aβ plaques after Maf1 knockout (FIGs. 8C-F).

### Example 5 Rescue of MEPSCs and LTP in hippocampal neurons by conditional knockout of Maf1

Synaptic plasticity is the ability of synapses to increase or decrease their response to neuronal activity over time. Many studies suggest that there are potential mechanisms of synaptic plasticity, including changes in the amount of neurotransmitters released presynaptically and the number of receptors on the postsynaptic membrane, thereby leading to the alteration of the efficiency of the postsynaptic cell's response to neurotransmitters. It is worth noting that LTP is one of the most commonly used techniques reflecting changes in synaptic plasticity in electrophysiological detection. In addition, mEPSC is a spontaneous synaptic activity, which refers to the shift of postsynaptic membrane current caused by the action of each vesicle on the postsynaptic membrane, reflecting the development and maturation of synapses. Previously, it was found that Maf1 knockdown could restore the morphology and structure of dendritic spines in APP/PS1 mice. To determine whether this restoration could reverse neurophysiological dysfunction, we further investigated changes in hippocampal synaptic plasticity.

Stereotactic injection of AAV-hsyn-GFP or AAV-hsyn-Cre-GFP was performed on Maf1-eCKO1-APP/PS1 mice. To detect the synaptic function, a whole-cell patch-clamp electrophysiology was used to measure mEPSCs (miniature excitatory postsynaptic currents) in brain slices, and a reduced frequency of mEPSCs was observed in these neurons of Maf1-eCKO1-APP/PS1 + AAV-hsyn-GFP vector mice, whereas the hippocampal neurons in Maf1-eCKO1-APP/PS1 + AAV-hsyn-Cre-GFP mice were recovered to WT levels (FIGs. 9A and 9B). The long-term potentiation (LTP) in the Schaffer collateral-CA1 pathway, a core cellular mechanism underlying learning and memory, was further detected.

Compared with wild-type mice, the LTP level of Maf1-eCKO1-APP/PS1 + AAV-hsyn-GFP vector mice was decreased and restored in Maf1-eCKO1-APP/PS1 + AAV-hsyn-Cre-GFP mice (FIGs. 9C and 9D). It is demonstrated that Maf1 knockout can restore hippocampal synaptic plasticity.

### Example 6 Validation of Maf1 regulating Grin1 and involvement in synaptic function and calcium signaling pathway

RNAseq experiments were performed to screen the related genes and signaling pathways altered after Maf1 knockdown in AD. The analysis showed that 884 differentially expressed genes were identified after Maf1 knockdown in AD, wherein 564 genes were downregulated and 320 genes were upregulated (FIGs. 10A-C).

GO enrichment analysis and KEGG pathway analysis of these different genes revealed that the downregulated genes were mainly associated with synapses, calcium ion signaling pathways, neuro-related ligand-receptor interactions, etc. The upregulated genes indicated that the upregulated genes were mainly associated with immune system diseases, complement and coagulation cascades.

In genes related to synaptic function and calcium signaling pathways, the Grin1 gene was found to have the best compliance (FIGs. 10D-F). The Grin1-encoded protein NMDAR1 is crucial for neuronal calcium homeostasis and synaptic remodeling. Therefore, these results indicate that Maf1 may regulate synaptic function by regulating Grin1 gene expression.

CUT&Tag experiments were further performed to explore the mechanism of Maf1 (FIGs. 11A and 11B). GO analysis suggested that synapses are associated with calcium ion binding (FIG. 11C), and KEGG indicated that genes potentially binding to Maf1 were closely related to the calcium signaling pathway and synaptic secretion of glutamine (FIG. 11D). By GO analysis and KEGG pathway analysis, it was suggested that 409 synapse-related genes and 74 synapse-related genes were enriched respectively; there were 30 genes, including Grin1, Grin2a, Grin2b, and Gria1, that intersected between them (FIG. 11E). We further analyzed the protein interaction network of these 30 genes, and notably, Grin1 was located at the core position (FIG. 11F), indicating that Maf1-NMDAR1 might regulate synaptic function by regulating calcium homeostasis in AD.

### Example 7 Maf1 regulates NMDAR1 transcription by binding to the promoter region of Grin1

Previous sequencing and biogenic analyses indicated that Maf1 might regulate calcium homeostasis and synaptic function by regulating Grin1 in AD. It was found that the level of NMDAR1 protein was downregulated after conditional knockout of Maf1 in hippocampal neurons (FIGs. 12A and 12B), which was also verified in primary neurons (FIGs. 12C and 12D).

To clarify a molecular mechanism by which Maf1 regulates structural remodeling of the neuronal dendritic spines through the Grin1-encoded protein NMDAR1 so as to affect the cognitive function, the protein interaction between Maf1 and NMDAR1 was first verified by a Co-IP (co-immunoprecipitation) experiment. The co-IP experiment showed that no co-IP of Maf1 and the NMDAR1 protein from hippocampal neurons was found, proving that there was no interaction between Maf1 and the NMDAR1 protein in hippocampal neurons (FIGs. 12E and 12F).

In view of the gene regulatory role of Maf1 as a transcription factor, the interaction between Maf1 and the Grin1 gene in the nucleus was further verified. To screen the binding sites of Maf1, four primers for the 2000 bp upstream region of the Grin1 gene were designed in the CH-IP (chromatin immunoprecipitation) experiment (as shown in FIG. 10. chromatin immunoprecipitation (Ch-IP)). The results of Ch-IP-qPCR confirmed the potential binding site of Maf1 in the promoter region of Grin1 (FIG. 12G).

Subsequently, a dual-luciferase reporter gene assay was performed to verify that Maf1 promotes Grin1 transcription. According to the description of Ch-IP, Maf1 NC and overexpression plasmids were constructed, Grin1 promoter plasmids were constructed, and Grin1 promoter sites were mutated, so as to obtain MT, MT1, MT2 and MT3 (FIG. 12H). In addition, the luciferase reporter plasmid was used to detect the activity of the Grin1 promoter in 293T cells.

The overexpression of Grin1 promoter WT and Maf1 enhanced the activity of the luciferase reporter gene, suggesting that Maf1 can interact with the Grin1 promoter and exert an enhanced regulatory effect at specific sites of the Grin1 promoter (FIG. 12I-L). It was found that the activity of the luciferase reporter gene with the mutant Grin1 promoter was not affected by Maf1 activation (FIG. 12I-L), and there was no difference in the activity of the luciferase reporter gene among the four mutant plasmids of the Grin1 promoter, suggesting a synergistic effect among the three sites on the Grin1 promoter.

The results of CH-IP analysis and dual-luciferase reporter gene assay indicated that Maf1 promoted Grin1 transcription by binding to the Grin1 promoter.

### Example 8 Grin1 promoter mutation attenuates the morphological effects of Maf1 knockdown

To further confirm the specificity of Maf1 affecting neuronal dendritic spines by regulating the Grin1 promoter, a "rescue" experiment was conducted using the Grin1 promoter mutant vector. For this purpose, hippocampal neurons were transfected with shSCR and shMaf1 plasmids carrying GFP fluorescence and wild-type or mutant Grin1 promoter plasmids without GFP, respectively (FIG. 13A), to analyze the morphology and number of dendritic spines of the neurons. Experimental results showed that compared with the SCR group, the shMaf1 + Grin1WT group exhibited a significant increase in the density of neuronal dendritic spines and a significant increase in the number of mature dendritic spines (FIGs. 13B and 13C), which was consistent with the phenotype of Maf1 in the gene knockout mice hereinabove. Compared with the shMaf1 + Grin1WT group, the dendritic spine density of neurons in these four groups was significantly reduced, wherein either the three sites of the Grin1 promoter were mutated separately (MT1/2/3) or mutated as a whole (MT), and the number of mushroom dendritic spines was also decreased (FIGs. 13B and 13C), indicating that there is a synergistic effect among the three binding sites and Maf1 is involved in the regulation of dendritic spines through the Grin1 gene.

Maf1-NMDAR1 signaling pathway regulated neuronal calcium homeostasis and a novel mechanism involved in AD synaptic remodeling. In the pathological state of AD, the expression of the transcriptional regulatory protein factor Maf1 was increased. By binding to the promoter region of Grin1, Maf1 regulated the expression of NMDAR1, further regulated calcium homeostasis, and altered the morphological structure and quantity of dendritic spines, thereby affecting synaptic function. Ultimately, it regulated synaptic remodeling and affected learning and memory functions (FIG. 14).

### Example 9 Screening of compounds binding to Maf1 protein

### Screening method:

SPR single-concentration detection technology: A surface plasmon is an electromagnetic wave on a metal surface, which is generated by interaction between freely oscillating photons and electrons. Surface plasmon resonance (SPR) is an optical phenomenon occurring at the interface between two media, where this phenomenon can be induced by photons or electrons. When light is incident from an optically denser medium to an optically rarer medium, total reflection occurs, generating an evanescent wave that enters the optically rarer medium. When the evanescent wave of total reflection meets the surface plasmon wave of the metal, resonance may occur, the reflected light energy decreases, and a resonance peak appears on the reflected light energy spectrum. This resonance is called surface plasmon resonance. An incident angle that causes surface plasmon resonance is called an SPR angle. An SPR biosensor provides a sensitive, label-free detection technique for real-time monitoring of molecular interactions. The sensor detects a change in the SPR angle, wherein the SPR is related to the refractive index on the metal surface. When an analyte binds to the chip surface, it causes a change in the refractive index of the chip surface, thereby inducing the change in SPR angle. This is the fundamental principle of the SPR biosensor for real-time detection of molecular interactions. In the interaction analysis, the change in SPR angle is recorded in real time on a sensing graph. A target protein-analyte interaction determination model is established by using surface plasmon resonance technology and the analyte is determined. The protein was immobilized using an amino coupling method, wherein the Maf1 protein antibody was diluted into a plurality of concentrations in a 96-well plate, and interacted with the target protein from low concentration to high concentration via the chip. The flow rate was 30 µL/min, and the duration was 150 s. The stock solution of the test substance was prepared with dimethyl sulfoxide (DMSO) to 10 mM, and diluted to 100 µM with PBS (5% DMSO) in a 96-well plate before testing, followed by sequential interaction with the protein. After injection at each concentration, the chip was regenerated for 5 min using a 10 mM glycine hydrochloride (pH 2.0) solution, and this process was repeated until all corresponding concentrations of the analytes were completed. Sample data were collected using BIAcore T200 Control software (v.2.0, GE Healthcare), and data of the reference channel were subtracted from the sample data. The data were globally fitted to a 1:1 Langmuir binding model using BIAcore T200 evaluation software (v.2.0, GE Healthcare), wherein the analyte in single-concentration detection obtained signal values in RU, and the analyte in multi-concentration detection obtained association and dissociation constants.

### Screening results:

According to SPR affinity detection, we screened out 8 compounds that were actually able to bind to the Maf1 protein.

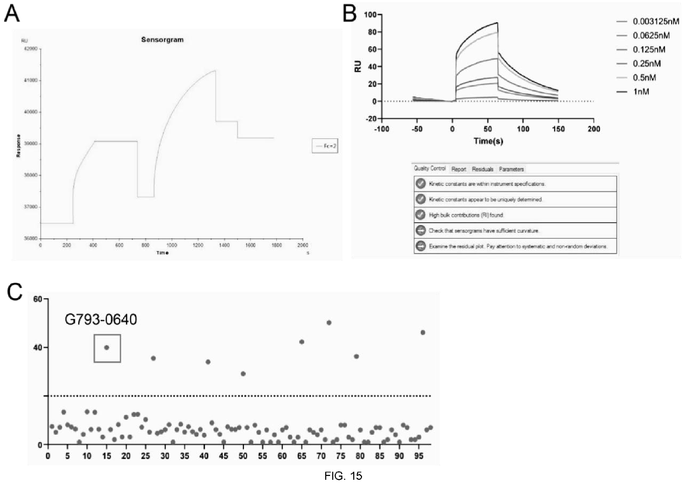

FIG. 15A: a conjugation diagram of a Maf1 protein; FIG. 15B: an interaction assay between a Maf1 protein and a Maf1 protein antibody (top: an interaction sensing diagram; bottom: quality control); FIG. 15C: a single-point screening results diagram.

### Example 10 Pharmacokinetic validation of compounds

**Compound preparation:** A compound of Formula I was commercially available, which was dissolved and diluted with a solution comprising 10% DMSO+90% (20% HP-β-CD).

| Compound of Formula I | Concentration | Dilution concentration | Peak region | Total measurement | Metered concentration | Correction |
|---|---|---|---|---|---|---|
| | mg/mL | | | ng/mL | mg/mL | % |
| Standard | 2.5 | DMSO | 628950.4 | 50.0 | **/** | **/** |
| Solution (IP) | 0.25 | DMSO | 58755.30 | 4.7 | 0.234 | 93 |

**Research method**: first, Balb/c strain mice were administered with a single intraperitoneal injection at a concentration of 2.5 mg/kg, and the content of the compound in serum was detected at 0.25, 0.5, 1, 2, 4, 6, 8 and 24 h after injection; second, brain tissues of mice at 0.5, 4 and 24 h after injection were collected to detect the compound content, so as to evaluate the blood-brain barrier penetration rate.

### Detection of blood drug concentration in mice after single administration

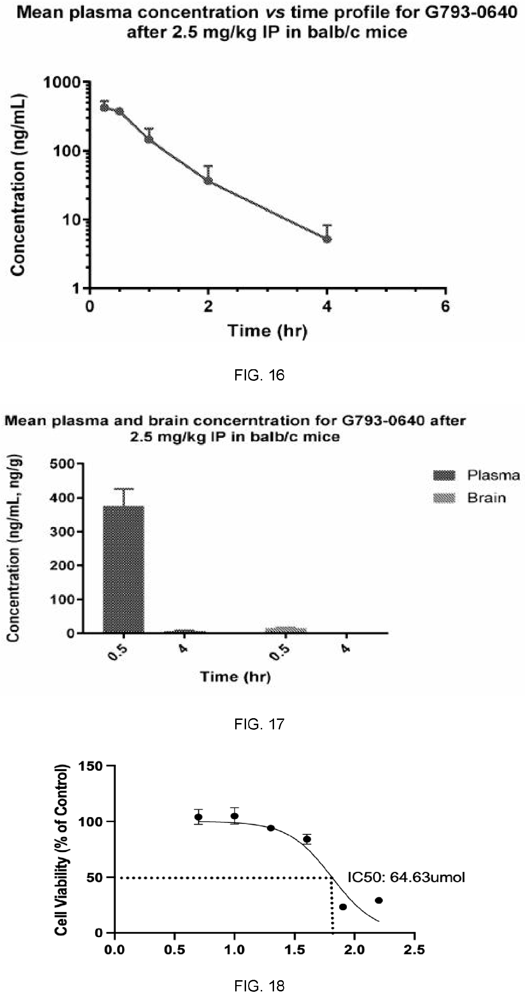

The table below shows the changes in the average blood concentration of the compound of Formula I in mice over time after intraperitoneal injection at a concentration of 2.5 mg/kg. The detection results indicated that the blood concentration of the compound gradually decreased at 0.25, 0.5, 1, 2 and 4 h after a single injection, and was undetectable in the animal body after 6 h (see FIG. 16).

### Blood concentration-time data after intraperitoneal injection in mice

BLOQ = Below LOQ, LOQ = 2.0 ng/mL

| | | | Animal_ID | | | | | | | | | | | |
|---|---|---|---|---|---|---|---|---|---|---|---|---|---|---|
| | | | 1 | 2 | 3 | 4 | 5 | 6 | 7 | 8 | 9 | Mean | SD | CV% |
| Dose (mg/kg) | Injection method | Time | Concentration | | | | | | | | | | | |
| | | (hr) | (ng/mL) | | | | | | | | | | | |
| 2.5 | IP | 0.25 | 369 | 350 | 551 | | | | | | | 423 | 111.2 | 26.3 |
| | | 0.5 | 364 | 328 | 431 | | | | | | | 374 | 52.7 | 14.1 |
| | | 1 | | | | 118 | 217 | 99.3 | | | | 145 | 63.2 | 43.6 |
| | | 2 | | | | 22.7 | 63.7 | 22.0 | | | | 36.1 | 23.9 | 66.1 |
| | | 4 | | | | 3.63 | 8.64 | 3.15 | | | | 5.14 | 3.04 | 59.2 |
| | | 6 | | | | | | | BLOQ | BLOQ | BLOQ | BLOQ | NA | NA |
| | | 8 | | | | | | | BLOQ | BLOQ | BLOQ | BLOQ | NA | NA |
| | | 24 | | | | | | | BLOQ | BLOQ | BLOQ | BLOQ | NA | NA |

### Test of drug content in brain tissue after single administration

The figure below (FIG. 17) shows the concentration of the compound of Formula I in the brain tissue of mice at 0.5, 4 and 24 h after intraperitoneal injection at a concentration of 2.5 mg/kg. The detection results indicated that the compound was detectable in the brain tissue of animals 0.5 h after a single injection, albeit at a low concentration, demonstrating that the small-molecule compound is capable of crossing the blood-brain barrier and entering the brain tissue.

### Drug concentration-time data in brain tissue after intraperitoneal injection in mice

BLOQ = Below LOQ, LOQ = 2.0 ng/mL

| | | | Animal_ID | | | | | | | | | | | | |
|---|---|---|---|---|---|---|---|---|---|---|---|---|---|---|---|
| | | | 1 | 2 | 3 | 4 | 5 | 6 | 7 | 8 | 9 | Mean | SD | CV% | Tissue /blood |
| Dose (mg/kg) | Injection method | Time | Concentration | | | | | | | | | | | | |
| | | (hr) | (ng/mL) | | | | | | | | | | | | |
| | | 0.5 | 16.8 | 10.3 | 14.4 | | | | | | | 13.8 | 3.28 | 23.8 | 0.037 |
| 2.5 | IP | 4 | | | | BLOQ | BLOQ | BLOQ | | | | BLOQ | NA | NA | NA |
| | | 24 | | | | | | | BLOQ | BLOQ | BLOQ | BLOQ | NA | NA | NA |

### Example 11: Toxicity assay of compounds

The CCK8 cell viability assay (CCK8) was adopted. Its principle is mainly based on the reduction reaction of WST-8 (chemical name: 2-(2-methoxy-4-nitrophenyl)-3-(4-nitrophenyl)-5-(2,4-disulfophenyl)-2H-tetrazolium monosodium salt). In the presence of living cells, WST-8 can be reduced by dehydrogenases in mitochondria to generate a highly water-soluble orange-yellow formazan product. The color intensity is directly proportional to the number of living cells, wherein the quantity of living cells can be indirectly reflected by measuring the absorbance of formazan products (typically at a wavelength of 450 nm). The CCK-8 assay is widely used in cell proliferation assay, cytotoxicity assay and tumor drug sensitivity test.

In in vitro experiments, the compound stock solution was prepared into 10 mM with DMSO and diluted with a culture medium before use. The 293T cell line was used for preliminary concentration screening of the compound, wherein concentration gradients were set as follows: 5 µmol, 10 µmol, 20 µmol, 40 µmol, 80 µmol and 160 µmol. After the cells were treated with the compound for 24 h, the CCK8 detection kit was used, wherein the cell viability was calculated based on the absorbance values detected by a microplate reader. Data processing was performed using GraphPad Prism software to plot the IC50 curve. The cell viability is shown in the table below:

| Concentration (µmol) | Duplicate hole 1 (%) | Duplicate well 2 (%) | Duplicate hole 3 (%) |
|---|---|---|---|
| 5 | 96.5645997 | 106.590738 | 109.195293 |
| 10 | 108.551159 | 109.839428 | 96.5365954 |
| 20 | 93.1198609 | 95.5563812 | 93.4559361 |
| 40 | 86.650484 | 86.7064938 | 78.8088099 |
| 80 | 22.74085 | 24.0011203 | 23.3569814 |
| 160 | 27.2218066 | 32.0108268 | 28.2580264 |

The IC50 curve of the compound of Formula I was plotted, as shown in FIG. 18.

Considering that it was required for identifying the pharmacological phenotype on neurons in the next step, primary neurons extracted from mouse brain tissues were further used for preliminary concentration exploration of the compound.

Primary hippocampal neuron culture: After ICR fetal mice at 18-21 days of gestation (E18-E21) were anesthetized, the fetal mice were dissected in a clean area and immersed in 75% ethanol for 1-2 min. The mouse head was taken out, the surface skull was removed with micro-scissors, and the brain tissue was transferred to an anatomical dissociation solution. Under a microscope, blood vessels and meninges were removed, hippocampal tissue was isolated, the extracted hippocampal tissue was placed in a 3.5 cm dish containing the anatomical dissociation solution, and the hippocampal tissue was cut into small fragments using micro-scissors. The minced hippocampal tissue was transferred to a 7 ml EP tube using a pipette gun. The hippocampal tissue hereinabove was centrifuged at 1000 rpm for 3-5 min, and the anatomical separation solution was discarded. 0.5-1 ml of papain digestion solution was added to the EP tube hereinabove, and the EP tube was placed in a 37°C incubator for 30 min. 300 µL of Trituration buffer 300 (DNase) and 5 mL of complete medium were added, and the hippocampal tissue was gently pipetted more than 10 times. The cell suspension was allowed to stand for 3 min, and 3-4 ml of supernatant was gently aspirated into a 15 ml tube using a pipette. 3-4 ml of complete medium was added again, and the hippocampal tissue was gently pipetted more than 10 times. Step 8 was repeated, and 3-4 ml of supernatant was transferred to the same 15 ml tube again. The previous step was repeated again. The collected supernatant was centrifuged at 1000 rpm, the supernatant was discarded, and 5 ml of complete medium was added. The mixture was gently pipetted and suspended, and then centrifuged again at 1000 rpm for 5 min. The complete medium was added to resuspend the cells, and then a portion of the cells was aspirated for counting. Generally, when neuronal cells were planted in a 24-well plate, the concentration of the cell suspension was generally 2×10⁵/ml, and 500 µl of cells is inoculated per well. The resuspended neuron cells with a diluted ratio were seeded onto a glass slide treated with PDL, then the glass slide was placed in a 37°C incubator for 10 min, an appropriate amount of complete medium was added, and then the culture is continued in the incubator.

A compound concentration gradient was set as follows: 5 µmol, 10 µmol, 20 µmol, 40 µmol, 80 µmol and 160 µmol, wherein the compounds were added when primary neurons were cultured in vitro for 13 days; after the cells were treated for 24 h, and then the cell viability was calculated based on the absorbance value detected by a microplate reader by using a CCK8 detection kit and the CCK8 detection kit, data processing was performed in GraphPad Prism software, and an IC50 curve was plotted. The cell viability is shown in the following table:

| Concentration (µmol) | Duplicate hole 1 (%) | Duplicate well 2 (%) | Duplicate hole 3 (%) |
|---|---|---|---|
| 0 | 100 | 100 | 100 |
| 1.25 | 94.5385369 | 88.6768191 | 77.1036661 |
| 2.5 | 90.5991202 | 97.5607601 | 84.4856808 |
| 5 | 70.7453562 | 77.3536459 | 75.3126271 |
| 10 | 51.7869159 | 58.8154745 | 65.026217 |
| 20 | 37.1260205 | 40.3800018 | 35.5707948 |
| 40 | 23.1366113 | 48.1855497 | 36.6841442 |
| 80 | 4.02148702 | 3.40636913 | 5.36506468 |
| 160 | 4.64821301 | 3.32933941 | 5.2440497 |

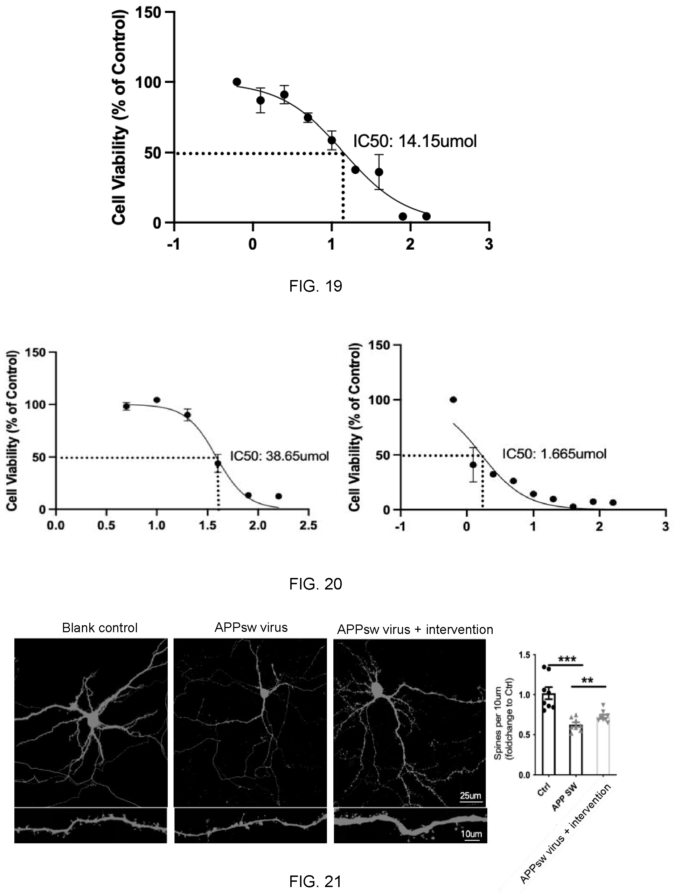

The IC50 curve was plotted, as shown in FIG. 19 below.

The compound of Formula II with an inhibitory effect on the MAF1 protein was subjected to CCK8 toxicity test on 293T and primary neurons, and IC50 curves were plotted respectively. The results showed that the IC50 values were 38.65 µmol (293T cell line) and 1.665 µmol (primary neurons), indicating that the concentration required to achieve half-lethal dose was low and the compound exhibited high toxicity. Therefore, further studies were not considered. As shown in the following figure (FIG. 20):

The structure of the compound of Formula II is as follows: 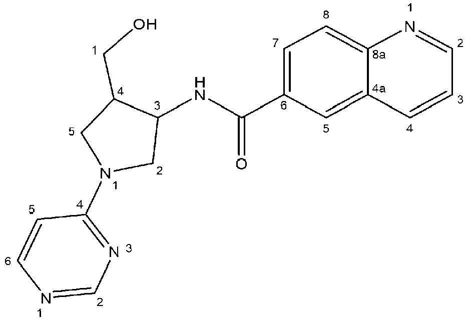 N-[4-(HYDROXYMETHYL)-1-(PYRIMIDIN-4-YL)PYRROLIDIN-3-YL]QUINOLINE-6-CARBOXAMIDE

### Example 12 In vitro efficacy experiment of compound

### Experimental method:

The extraction and culture of primary neurons were the same as those hereinabove (toxicity experiment section).

Transfection of neurons using plasmid calcium: A neuron culture medium (half medium change) was aspirated and placed in a 37°C water bath for later use, and Neurobasel A was added to a 24-well plate. 500 ng of plasmid DNA was dissolved in 12.5 µl of CaCl₂ solution, and then centrifuged. 12.5 µl of HBS was added to the EP tube, and then centrifuged. The mixed solution hereinabove was added to the 24-well plate. The 24-well plate was gently shaken, and then placed back into a 37°C incubator to stand for 90 min. Fresh Neurobasel A was aspirated to a new 15ml EP tube, CO₂ was perfused to saturate the Neurobasel A, the original medium in the 24-well plate was discarded after the color turned yellow, CO₂-saturated Neurobasel A was added, and the mixture was placed in a 37°C incubator to stand for 15 min. The culture medium was discarded, and the original medium and a fresh complete medium were added at a ratio of 1:1. The 24-well plate was placed into the incubator for further culture. After 24 h-48 h, the transfection status was observed under a microscope. Neuron cells were collected at DIV 14 and fixed with paraformaldehyde to observe dendritic spine changes.

The method for infecting neurons with lentiviruses is as follows:

Neurons were planted at a concentration of about 2.0×10⁴ cells/L, with 1 ml/well of cell culture medium, and cultured in a 37°C, 5% CO₂ incubator. On day 6 of in vitro culture (DIV6), the original medium was semi-quantitatively collected, and the lentivirus stock solution (100 µl) was diluted with the original medium (10 ml) at a ratio of 1:60-1:40. The diluted virus-containing medium was added to a 24-well culture dish at 1 ml/well. After neurons were infected with the viruses for 8-12 h, the cell culture medium containing lentiviruses was discarded, an equal volume (1 ml/well) of the collected original medium was added, and then the neurons were cultured in the incubator.

Primary neurons were transfected with calcium-transformed GFP plasmids on day 4 of *in vitro* culture to enable the neurons to carry green fluorescence for observing neuronal dendrites. On day 6, the neurons were transfected with APPsw lentiviruses to establish an *in vitro* model simulating Alzheimer's disease. The compound stock solutions were all prepared into 10 mM using DMSO. When the primary neurons were cultured in vitro for 13 days, the compounds were diluted to a concentration of 2.5 µmol using the culture medium and added to treat the cells for 24 h. After 14 days of in vitro culture, the neurons were fixed with 4% PFA, photographed under a 100× oil immersion lens of a confocal fluorescence microscope, and the dendritic spine density per 10 µm length of the neurons was calculated by observing GFP green fluorescence.

### Result analysis:

The green fluorescence images in the figure below (FIG. 21) are representative schematic diagrams of the three groups (the upper part shows the entire neuron, and the lower part shows the dendritic spine branches), and the right figure is a statistical diagram of the dendritic spine density of the three groups (normalized to the control group, n=8). The results showed that compared with the blank control, the total density of neuronal dendritic spines in the in vitro AD model (APPsw virus) was reduced, whereas the dendritic spine density was increased after compound intervention. The results indicated that the compound intervention increased the number of dendritic spines of neurons in the AD in vitro model and had an improving effect on synapses, as shown in FIG. 21:
wherein the number of dendritic spines of neurons in the statistical chart is shown in the following table (normalized to the control group):

| | Ctrl | APP SW | APP SW+intervention |
|---|---|---|---|
| Neuron-1 | 0.8572 | 0.5517 | 0.7841 |
| Neuron-2 | 0.8408 | 0.7470 | 0.7357 |
| Neuron-3 | 0.8011 | 0.6770 | 0.6856 |
| Neuron-4 | 1.0217 | 0.5948 | 0.8651 |
| Neuron-5 | 1.3451 | 0.5174 | 0.6806 |
| Neuron-6 | 1.3211 | 0.7204 | 0.6909 |
| Neuron-7 | 0.8939 | 0.6133 | 0.6481 |
| Neuron-8 | 1.0564 | 0.5881 | 0.7086 |

### Example 13 In vivo efficacy experiment of compound

### Experimental method:

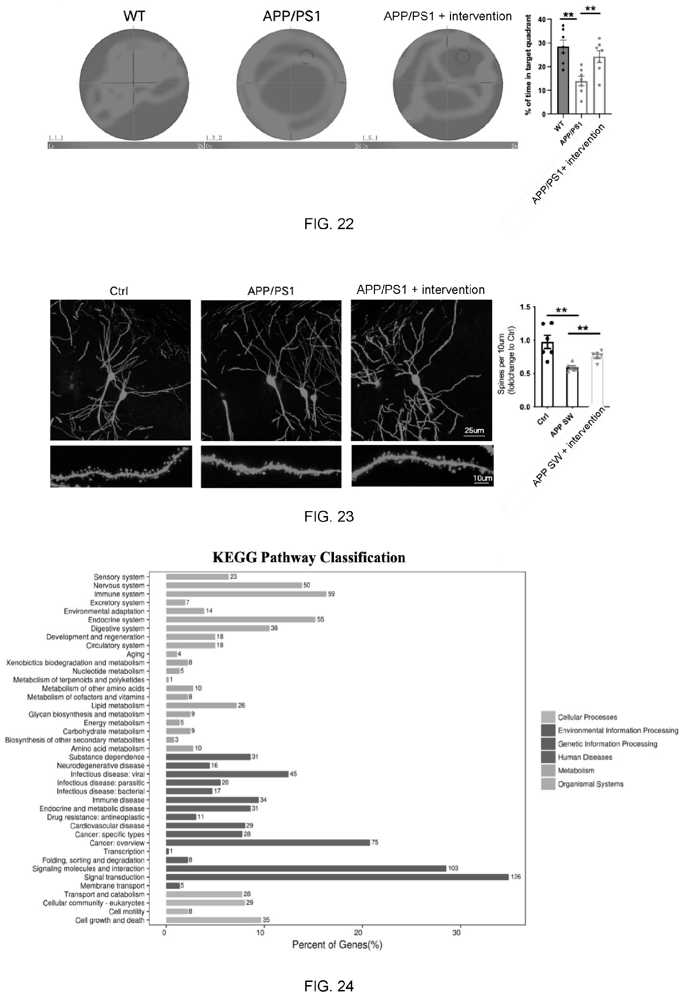

Thy1-GFP mice are commonly used in neuroscience research. The fluorescence labeling of GFP facilitates convenient observation and tracking of specific cell types, especially neurons. APP/PS1 transgenic mice (an animal model of Alzheimer's disease) were crossed with Thy1-GFP mice. The crossed mice aged 5 months were intraperitoneally injected with a compound solution at a concentration of 2.5 mg/kg for 1 month continuously. Subsequently, the crossed mice aged 6 months were subjected to behavioral test (Morris water maze MWM experiment) to evaluate learning and memory abilities (as shown in FIG. 22).

Water maze: A water maze experimental apparatus is circular stainless steel with a diameter of 100 cm and a height of 50 cm, wherein a pool is equally divided into 4 quadrants according to the 4 orientations of east, south, west and north, a circular black hidden platform with a diameter of 9 cm and a height of 27 cm is placed in the center of the target quadrant. In the acquisition training phase, the position of the hidden platform remained unchanged, and the water temperature was maintained at 22-24°C. The movement trajectory of the mouse was synchronously recorded by the camera directly above the water pool, and analyzed by the EthovisionXT monitoring and analysis system. Throughout the experiment, the visible platform test was performed on day 1. The subsequent 5 consecutive days of the hidden platform trial served as the acquisition phase, and day 7 was designated as the spatial probe trial phase. The mice were placed in a room where the water maze was located 1 day before the experiment to adapt to the environment. Hidden platform acquisition phase: The mice were trained twice daily at the same time, the same mouse entered the water from different quadrants each time, whereas different mice entered the water at the same position. The swimming duration was set to 60 s. Mice that found the platform and stayed on it for more than 5 s were regarded as having located the platform. For mice that failed to find the platform within 60 s, they were guided to the platform and allowed to remain there for 15 s. Space exploration test phase: On day 7, the platform in the pool was removed, the farthest point from the platform in a straight line was selected as the monitoring entry point, and the mouse was placed into the water at this location. The mouse swam in the water for 60 s, and the percentage of time the mouse spent in the target platform quadrant was recorded, representing the learning and memory ability of the mouse.

After the water maze behavioral test, the mice were anesthetized and perfused, and then brain tissue sections were taken. The density of neuronal dendritic spines was observed under a confocal fluorescence microscope using GFP green fluorescence.

### Result analysis:

FIG. 22 is a heatmap of swimming trajectories of mice in each group within 60 s on day 7 of the spatial exploration phase. From the heatmap, the trajectory diagram of the Alzheimer's disease animal model mice (APP/PS1) shows proximity to the wall edge. Through statistical analysis of the time percentage in the platform quadrant (upper right) among the three groups, compared with WT mice, the time percentage of APP/PS1 mice in the platform quadrant decreased, whereas the time percentage of mice in the platform quadrant was increased after compound intervention. The results showed that continuous injection of the compound was able to improve the learning and memory abilities of AD mice.

The numerical values of the statistical chart are shown in the following table:

| | WT (%) | APP/PS1 (%) | APP/PS1+ intervention (%) |
|---|---|---|---|
| Animal-1 | 32.445 | 9.778333333 | 12.16666667 |
| Animal-2 | 25.77667 | 14.55666667 | 26.78333333 |
| Animal-3 | 28.22 | 20.77333333 | 27.88833333 |
| Animal-4 | 37.33333 | 16.44333333 | 19.335 |
| Animal-5 | 21.33667 | 5.336666667 | 31.945 |
| Animal-6 | 18.555 | 12 | 22.00166667 |
| Animal-7 | 35.66667 | 18.49666667 | 29.11 |

FIG. 23 shows representative images of hippocampal neurons in brain slices from three groups of mice. The statistical analysis results indicated that compared with Ctrl, the total dendritic spine density of hippocampal neurons in AD mice was reduced, whereas the dendritic spine density was increased after compound intervention. As shown in FIG. 23, the right panel shows the statistical chart of dendritic spine densities in the three groups:

The numerical values of the statistical chart are shown in the following table (normalized to the control group):

| | Ctrl | APP SW | APP SW + intervention |
|---|---|---|---|
| Neuron-1 | 0.8211 | 0.6970 | 0.7721 |
| Neuron-2 | 1.0237 | 0.5358 | 0.7284 |
| Neuron-3 | 0.6752 | 0.5674 | 0.6276 |
| Neuron-4 | 1.2451 | 0.6237 | 0.7954 |
| Neuron-5 | 0.8239 | 0.5761 | 0.8425 |
| Neuron-6 | 1.2564 | 0.5587 | 0.7853 |

### Example 14 Transcriptome detection of hippocampal tissue after intervention with compound

After the water maze behavioral test, the mice in the AD group and the mice in the AD+compound intervention group were anesthetized and perfused, and hippocampal tissues were collected for transcriptomic sequencing to investigate the differentially expressed genes and involved signaling pathways in the compound intervention group compared with the AD group as the baseline. For the differentially expressed genes, further enrichment analysis and KEGG pathway analysis were performed. The results (as shown in FIG. 24) revealed that, compared with the transcriptional levels of genes in the hippocampus of AD mice, the differentially expressed genes with altered transcriptional levels after compound intervention were mainly involved in the nervous system, neurodegenerative diseases, and signal transduction. For example, in the Nervous system-related gene set, the transcriptional levels of 50 genes were altered, wherein in the Neurodegenerative disease-related gene set, the transcriptional levels of 16 genes were altered. Transcriptomic analysis suggests that the functional pathway of small molecule compounds binding to the target Maf1 protein is closely related to neural signal transduction.

## Claims

1. A marker for early diagnosis of Alzheimer's disease, wherein the marker is a Maf1 gene or a protein thereof, and the high expression of the Maf1 gene or the protein thereof in neuronal cells indicates that a test subject is a high-risk population for the Alzheimer's disease.

2. A reagent or kit for early diagnosis of Alzheimer's disease, wherein the reagent or kit comprises a reagent capable of detecting the expression of a Maf1 gene or a protein thereof.

3. Use of a Maf1 gene or a protein thereof in the preparation of a product for early diagnosis of Alzheimer's disease, wherein the use is to determine whether a patient is at a high risk of the Alzheimer's disease based on the expression of the Maf1 gene or the protein thereof in neuronal cells.

4. The use of the Maf1 gene or the protein thereof in the preparation of the product for early diagnosis of Alzheimer's disease according to claim 3, wherein when the Maf1 gene or the protein thereof is highly expressed in the neuronal cells, the test subject is determined to be at a high risk of the Alzheimer's disease.

5. An inhibitor for inhibiting Maf1 protein expression, wherein the structure of the inhibitor is as follows:

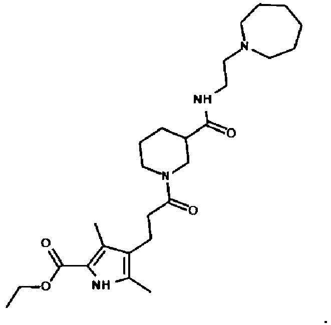

6. The inhibitor for inhibiting the Maf1 protein expression according to claim 5, wherein the inhibitor comprises, but is not limited to, chemically synthesized substances, naturally occurring compounds and/or biosynthesized substances.

7. A promoter for promoting the expression of a Maf1 protein.

8. Use of a promoter for promoting the expression of a Maf1 gene or a protein thereof in the preparation of an animal model, wherein the promoter is an experimental preparation.

9. Use of the inhibitor for inhibiting the expression of the Maf1 gene or the protein thereof according to claim 5 in the preparation of a medicament for treating AD disease.

10. A pharmaceutical preparation for treating AD disease, wherein the pharmaceutical preparation comprises a substance that inhibits the expression of a Maf1 gene or a protein thereof, and
the structure of the substance is as follows:
